# EUROPEAN PATENT APPLICATION

(11) **EP 2 316 970 A2**
(43) Date of publication of application: **04.05.2011**
(21) Application number: 09764040.3
(22) Date of filing: 06.07.2009
(51) Int. Cl.: C12Q 1/68

(54) **METHODS FOR THE TREATMENT AND DIAGNOSIS OF CANCER**

(30) Priority: 04.07.2008 ES 200802007
(71) Applicant: Traslational Cancer Drugs Pharma, S.L., 28004 Madrid (ES)
(72) Inventor: LACAL SANJUÁN, Juan Carlos, E-28250 Torrelodones - Madrid (ES); RAMÍREZ DE MOLINA, Ana, E-28224 Pozuelo de Alarcón - Madrid (ES); GALLEGO ORTEGA, David, E-28029 Madrid (ES)
(74) Representative: ABG Patentes, S.L.
(86) International application number: PCT/IB2009/052936
(87) International publication number: WO 2010/001369

(57) **Abstract**

The present invention relates to methods for the treatment of cancer based on the induction of the choline kinase beta (hereinafter ChoKβ) activity as well as to methods for the design of personalized therapies and for determining the response of an agent capable of inducing choline kinase beta (hereinafter ChoKβ) for the treatment of cancer as well as to methods for determining the prognosis of a patient based on the determination of the ChoKβ expression levels as well as based on the determination of the relationship between the ChoKβ and ChoKα expression levels. Finally, the invention relates to methods for determining the response of a patient who suffers from cancer to ChoKα-inhibiting agents based on the determination of the PEMT and/or ChoKp expression levels.

## Description

### Technical Field of the Invention

The present invention relates to methods for the treatment of cancer and, in particular, to methods for the treatment of cancer based on the induction of choline kinase beta (hereinafter ChoKβ) activity as well as to methods for the design of personalized therapies and for determining the response to an agent capable of inducing choline kinase beta (hereinafter ChoKβ) for the treatment of cancer as well as to methods for determining the prognosis of a patient based on the determination of ChoKβ expression levels as well as based on the determination of the relationship between ChoKβ and ChoKα expression levels. Finally, the invention relates to methods for determining the response of a patient who suffers from cancer to ChoKα inhibiting agents based on the determination of the PEMT and/or ChoKβ expression levels.

### Background of the Invention

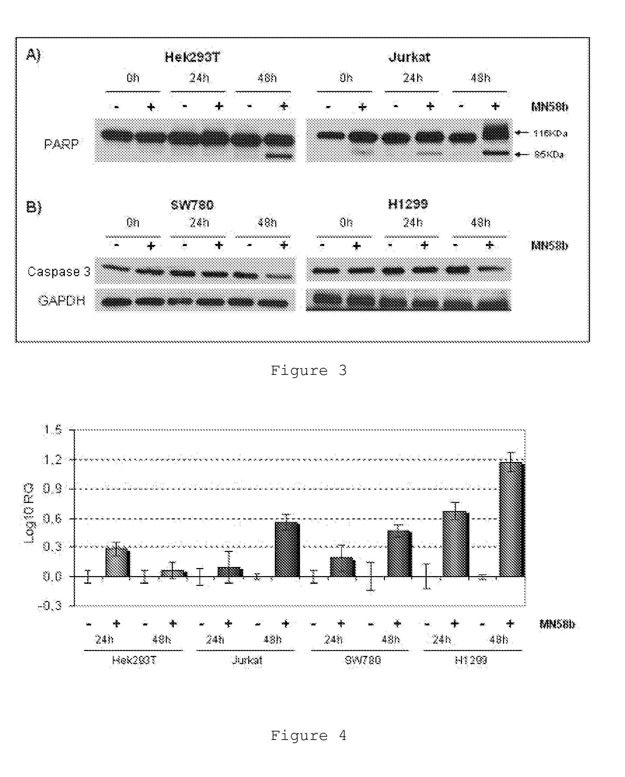

Choline kinase (ChoK) is the first enzyme in the so-called Kennedy pathway of phosphatidylcholine (PC) biosynthesis, which is the major lipid of the membranes of eukaryotic cells. In particular ChoK catalyzes the reaction of transformation of choline (Cho) into phosphocholine (PCho) using a molecule of ATP and Mg²⁺ as a cofactor. The Kennedy pathway continues with the enzyme action on PCho of the CDP-phosphocholine cytidyltransferase (CT) originating CDP-choline, and subsequently of the DAG-choline phosphotransferase (CPT) resulting in PC (Figure 3). Although the ChoK activity forms the first step in PC synthesis, it is considered that the limiting or regulating step of PC biosynthesis is the one catalyzed by CT.

The amino acid sequence forming the choline kinase domains are highly conserved in all eukaryotic organisms, the homology between murine and human genes being 85-88% for example. In mammals, the choline kinase family is encoded in two different genes: CHKA and CHKB, located in humans in chromosomes 11q13.2 and 23q13.33 respectively (Ensembl Genome Browser v48, Gene view: http://www.ensembl.org/). Due to their high homology, their occurrence because of a gene duplication process and subsequent divergence from a common ancestor has been suggested. The expression of these genes results in the translation of three proteins with choline/ethanolamine kinase domain: ChoKα1, ChoKα2 and ChoKβ1 (previously referred to as *ChoK-like).* The alpha isoform has two variants generated by alternative splicing of the primary mRNA: ChoKα1 of 457 amino acids (aa), and ChoKα2 (439 aa), from which it differs in only 18 aa in the N terminal region. The beta isoform also has two different alternative splicing variants, only one of which, ChoKβ1, has kinase activity. ChoKβ1 has 395 aa and differs from the alpha isoform in approximately 40% of its sequence (Aoyama *et al.,* 2004) (Figure 4). Finally, a reading frame shift in the ChoKβ transcript results in the occurrence of ChoKβ2, a shorter protein (127 aa) which lacks choline/ethanolamine kinase domain, and which differs from the variant 1 in its C-terminal end. The role that this variant can have is not known, however, a murine variant of 164 aa with similar characteristics referred to as ChoKα3 has been identified.

In addition to its role in lipid metabolism, there is considerable evidence which indicates that ChoK is involved in carcinogenesis. The first evidence that ChoK could play an important role in carcinogenesis arose from the observation that an increase of PCho occurred during cell transformation mediated by the RAS oncogene. Later it was demonstrated that the increase of PCho was caused by an increase of ChoK activity (Ramirez de Molina et al., 2001, Biochem. Biophys. Res. Commun. 285:873-879), mediated by two of the most known effectors of the RAS oncogene, PI3K and Ral-GDS (Ramirez de Molina et al., 2002, Oncogene 21: 937-946.). The production of PCho as an essential process in cell growth induced by growth factors both in murine fibroblasts and in different systems of human cells, in which treatment with ChoK specific drugs results in a blocking of DNA synthesis induced by different factors such as EGF, PDGF or HRG, has also been described.

ChoK is overexpressed in a high percentage of cell lines derived from human tumors as well as in different human breast, lung, colon, bladder and prostate tumor tissue. (Nakagami et al., 1999, Jpn. J. Cancer Res 90:419-424; Ramirez de Molina et al., 2002, Oncogene 21: 4317-4322; Ramirez de Molina et al., 2002, Biochem Biophys. Res. Commun. 296: 580-583). These tumor types represent more than 70% of the total of cases of cancer in developed countries. Biochemical data show an activation of the enzyme in a high percentage of cases, an increase of ChoK in tumor conditions both at the transcriptional and post-translational levels being put forward (Ramirez de Molina *et al*., 2002a). The incidence of overexpression or overactivity of ChoK in these tumor types is generally very high, ranging from 40 to 60% (Ramirez de Molina et al., 2004, Cancer Res 64: 6732-6739; Ramirez de Molina et al., 2002, Biochem Biophys Res Commun 296:580-583). In the cases which were analyzed, an association between ChoKα activation and degree of malignancy stands out (Ramirez de Molina et al., 2002, Oncogene 21: 4317-4322). Finally, a study has recently been conducted with 167 samples of patients with non-small-cell lung cancer (NSCLC), which shows that the patients whose tumors demonstrate high ChoKα expression significantly have a worse prognosis of the disease, which could have important clinical consequences (Ramirez de Molina et al., 2007, Lancet Oncol 8:889-897). All these studies have been conducted for the α isoform.

Considerable evidence demonstrates the alteration of ChoK in the carcinogenic process, indicating this enzyme as a target to develop an antitumor strategy based on the specific inhibition of its activity. First, different *in vitro* studies in cells transformed by oncogenes demonstrated the existence of a high correlation between the inhibition of the enzyme with the inhibition of the cell proliferation without having the lethality of hemicholinium-3 associated thereto(Campos et al., 2000, Bioorg Med Chem Lett 10: 767-770.; Cuadrado et al., 1993, Oncogene 8: 2959-2968; Hernandez-Alcoceba et al., 1997, Cancer Res 59: 3112-3118; Jimenez et al., 1995, J Cell Biochem 57: 141-149.). *In vivo* cell growth inhibition assays in xenotransplants of human tumor cells of epidermoid carcinoma, colon adenocarcinoma and breast adenocarcinoma generated in athymic mice have also been successfully conducted (Hernandez-Alcoceba et al., 1999, Cancer Res 59: 3112-3118; Lacal, 2001, IDrugs 4: 419-426; Ramirez de Molina et al., 2004, Cancer Res 64:6732-6739). Finally, the specificity of MN58b on its target ChoK has been demonstrated *in vivo* in xenotransplants of both breast and colon cancer cells by means of NMR, whereby it was determined that only the levels of PCho, but not of other phosphomonoesters, were affected after antitumor treatment with MN58b (Al-Saffar et al., 2006, Cancer Res 66: 427-434).

Nevertheless, despite thorough knowledge of the mechanisms of action of ChoKα inhibitors, it is still unknown if ChoKβ can be used as a target for the development of antitumor drugs or if said enzyme can be used as a biomarker for response to antitumor drugs or for prognosis in patients who suffer from cancer.

### Summary of the Invention

In a first aspect, the invention relates to a method for determining the prognosis of a patient suffering from cancer comprising determining the ChoKβ expression levels in a sample of said patient in which reduced levels of ChoKβ in relation to the levels in a reference sample are indicative of the patient showing a poor prognosis.

In a second aspect, the invention relates to a method for determining the prognosis of a patient suffering from cancer comprising determining the ChoKα and ChoKβ expression levels in a sample of said patient in which reduced levels of ChoKα and high levels of ChoKβ in relation to the expression levels of said proteins in a reference sample are indicative of the patient showing a good prognosis.

In a third aspect, the invention relates to a method for determining the response of a patient with cancer to the treatment with a ChoKα inhibitor comprising determining in a sample of said patient the expression levels of a protein selected from the group of PEMT and ChoKβ, in which an increase of the PEMT expression levels or an increase of expression of the levels of ChoKβ in relation to the levels in a reference sample are indicative of a good response to the ChoKα inhibitor.

In a final aspect, the invention relates to a ChoKβ activity-inducing agent for its use in the treatment of cancer.

### Description of the Drawings

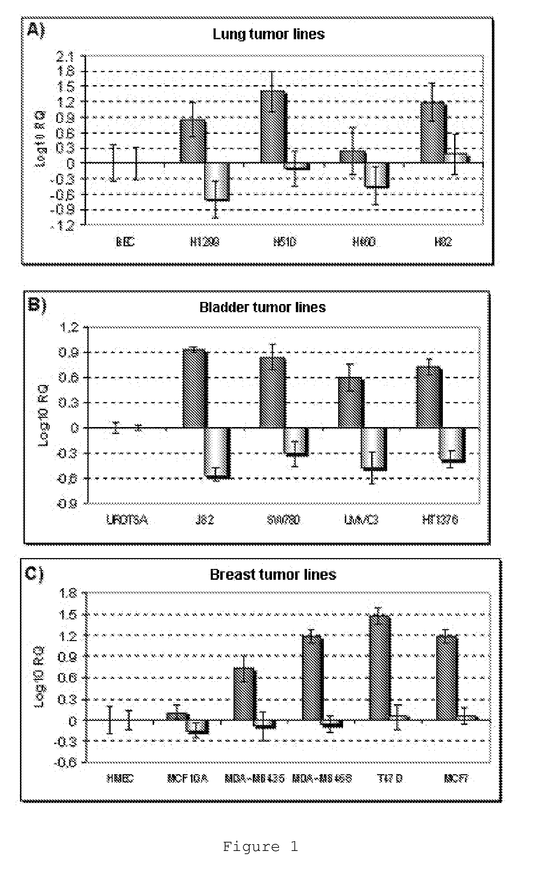

Figure 1. The mRNA levels of Chokα are increased in the tumor lines whereas those of Chokβ are unchanged or are reduced. Results of the quantitative PCR of Chokα (gray) and Chokβ (white) in human tumor lines of: A) lung, B) bladder and C) breast. The mRNA levels of tumor lines are compared with a normal epithelial line of the same origin by means of the 2^{-ΔCt} method. The endogenous gene for normalization used was 18S.

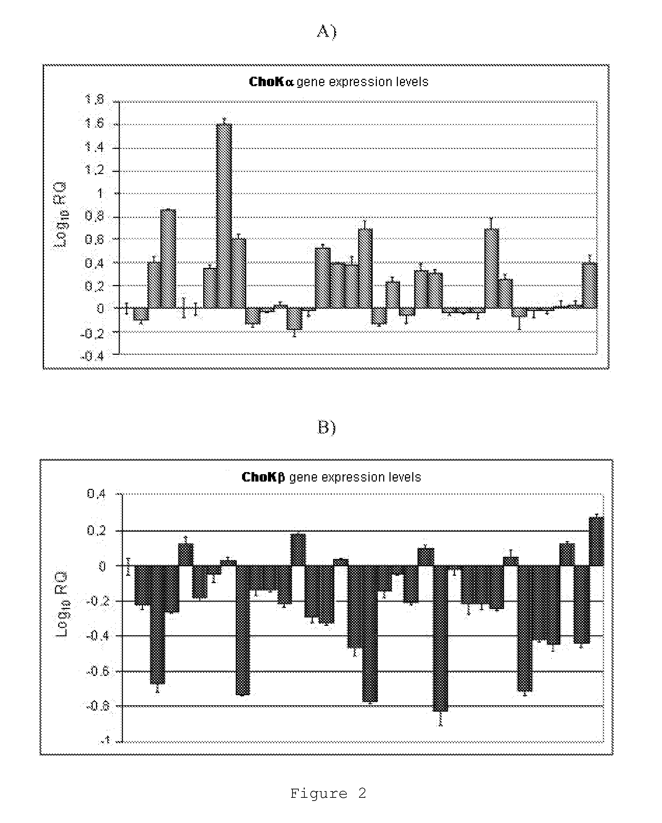

Figure 2. Pattern of gene expression of the Chokα and Chokβ isoforms in samples of patients diagnosed with non-small-cell lung cancer. mRNA expression levels of Chokα (A) or Chokβ (B) in lung tumor samples compared with a commercial normal lung tissue using the 2^{-ΔCt} method. The results correspond to the Log10 RQ (relative quantity) of the α or β isoforms with respect to the expression of the endogenous gene (18S). The expression of the normal tissue is shown in the first column in each case.
Figure 3. Induction of apoptosis in response to MN58b. Hek293T, Jurkat, SW70 and H1299 cells were treated with 20 µM of MN58b for 0h, 24h and 48h, and the same cells were maintained untreated for the same time period as a control. Cell extracts of these lines were resolved by PAGE-SDS and transferred to a nitrocellulose membrane for their immunodetection with antibodies. Examples of photographs are shown as a result of the immunodetection in different cell lines of A) PARP and B) Caspase 3, the degradation of which is an indicator of apoptosis. GAPDH was used as load control.
Figure 4. Increase of the transcription of Chokβ in response to MN58b. Hek293T, Jurkat, SW70 and H1299 cells were treated with 20µM of MN58b for 24h and 48h, and the same cells were maintained untreated for the same time period as a control. The total RNA of said cells was then extracted and quantitative PCR was performed. A response of an increase of mRNA levels of Chokβ was obtained in all the evaluated cases in response to the drug. Log₁₀RQ obtained by the 2^{-ΔΔCt} method is depicted, the RQₘₐₓ - RQₘᵢₙ interval being the error.

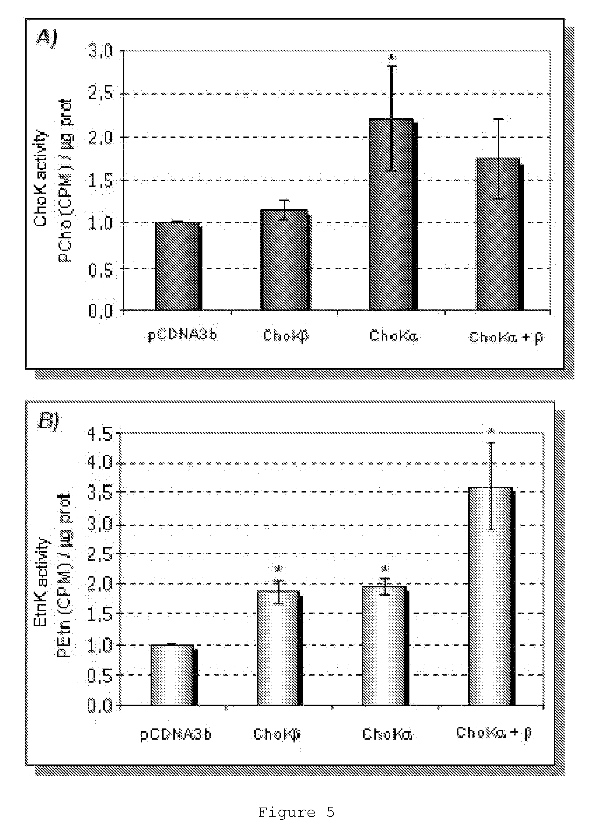

Figure 5. The coexpression of Chokα and Chokβ causes opposite effects in the intracellular ethanolamine and choline levels. Hek293T cells were transfected with the expression vectors of Chokα, Chokβ, Chokα/Chokβ at the same time or the empty pCDNA3b vector. The cells were labeled at equilibrium with 14C-choline or 14C-ethanolamine for 24h. The lipids were extracted. The quantity of intracellular PEtn or PCho with respect to total lipids is depicted. The joint overexpression of Chokα and Chokβ reduced the levels of PCho reached with the overexpression of Chokα separately, whereas an increase in the intracellular levels of PEtn occurred. The results which are shown correspond to the mean ± SEM of 3 independent experiments performed in triplicate. * Statistically significant variations (p< 0.05).

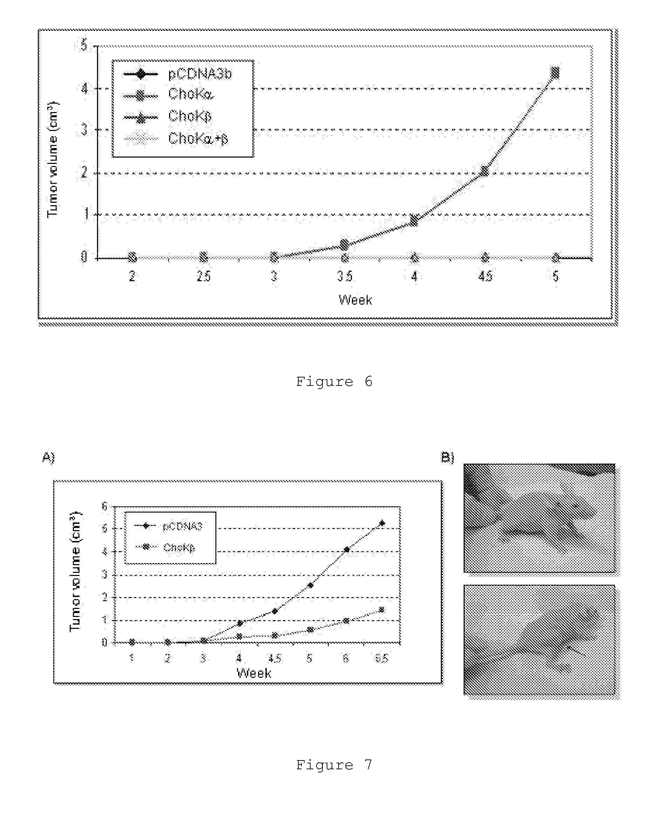

Figure 6. Chokβ inhibits the oncogenic capacity of Chokα. Hek293T cells were transfected with the expression vectors of Chokα, Chokβ, both (Chokα/Chokβ) or the empty pCDNA3b vector as negative control. After transfection, 10⁶ cells were subcutaneously inoculated in the back of athymic mice (nu⁻/nu⁻). It was found that the generation of tumors in the case of Chokα is statistically significant (p≤ 0.001). The promotion of tumors caused by Chokα was completely eliminated when Chokβ is overexpressed at the same time.
Figure 7. Chokβ inhibits the oncogenic capacity of Chokα (II). ADJ cells from tumors generated by the overexpression of Chokα were transfected with the expression vectors of Chokβ or the empty pCDNA3b vector as negative control. After transfection, 10⁶ cells were subcutaneously inoculated in the back of athymic mice (nu⁻/nu⁻). A) Comparison of the growth of the tumors generated by ADJ/Chokβ and ADJ/pCDNA3b, B) Photographs of the xenografts occurring in athymic mice injected with ADJ/Chokβ (upper panel) and ADJ/pCDNA3b (lower panel) cells (week 4.5).

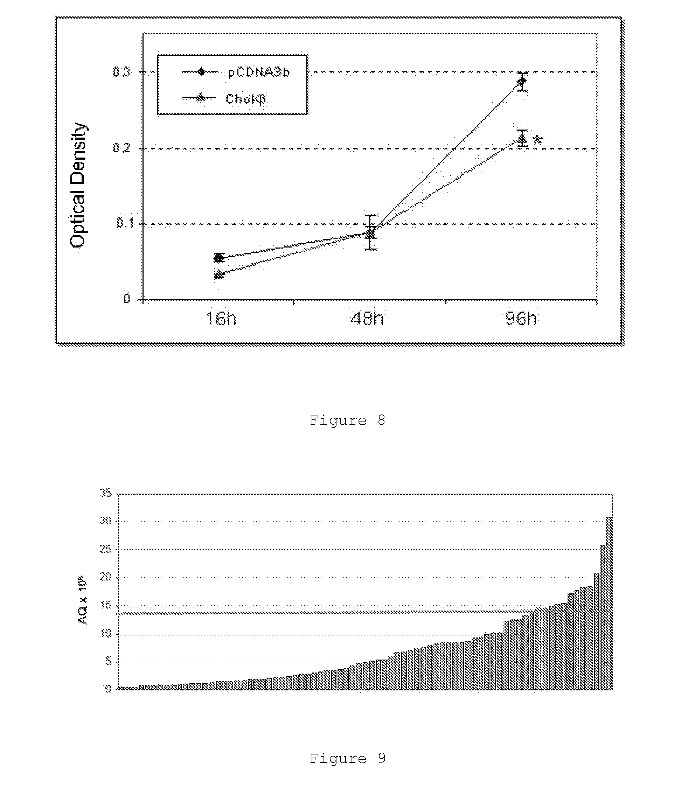

Figure 8. The overexpression of Chokβ in ADJ cells derived from Hek293T delays cell proliferation. ADJ cells stable for the expression of Chokα were transfected with the expression vectors of Chokβ or the empty pCDNA3b vector as negative control. The cells were seeded in 24-well plates at a density of 10⁴ cells per well and were incubated for 16, 48 and 96 hours in optimal growth conditions, the optical density being measured after staining with crystal violet. The growth of the cells transfected with Chokβ is significantly lower after 96h. The statistical significance considered is p≤ 0.05, marked with an asterisk.
Figure 9. Quantification of ChoKβ expression in tumor samples of patients with NSCLC and compared with the expression in commercial normal tissue used as a reference.

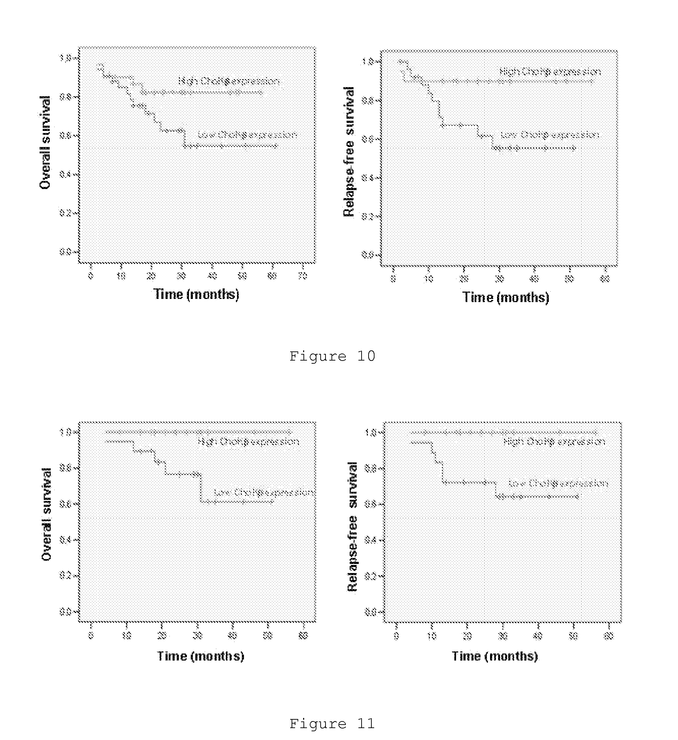

Figure 10. Kaplan-Meier plots for ChoKβ expression and overall and relapse-free survival in patients with NSCLC.
Figure 11. Kaplan-Meier plots for ChoKβ expression and survival in patients who had stage I NSCLC.

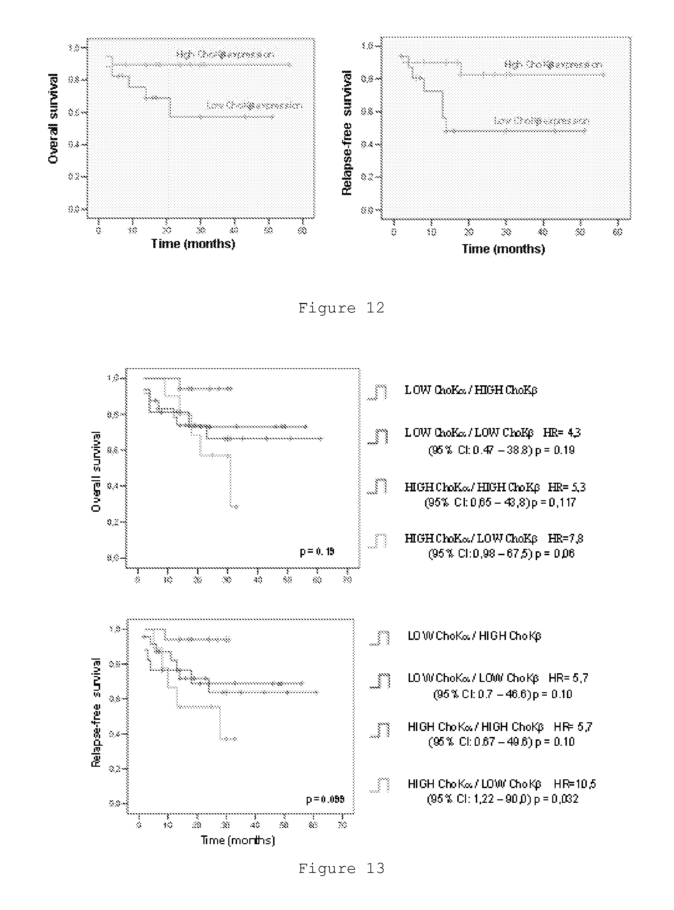

Figure 12. Kaplan-Meier plots for ChoKβ expression and survival in patients with squamous cell carcinoma.
Figure 13. Kaplan-Meier plots for the combined effect of ChoKα and ChoKβ expression on the survival of patients with NSCLC.
Figure 13. Increase of the transcription of PEMT in response to MN58b. Hek293T, Jurkat, SW780 and H1299 cells were treated with 20µM of MN58b for 24h and 48h, and the same cells were maintained untreated during the same time periods as a control. The total RNA of said cells was then extracted and quantitative PCR was performed. Log₁₀RQ obtained by the 2^{-ΔΔCt} method is depicted, the interval RQₘₐₓ - RQₘᵢₙ being the error. The arrow in the case of the Hek293T and SW780 cell lines as the error bar indicates that the control does not have PEMT expression, and that it starts to be expressed in the treated cells, therefore the comparison is extrapolated to the maximum number of PCR cycles.

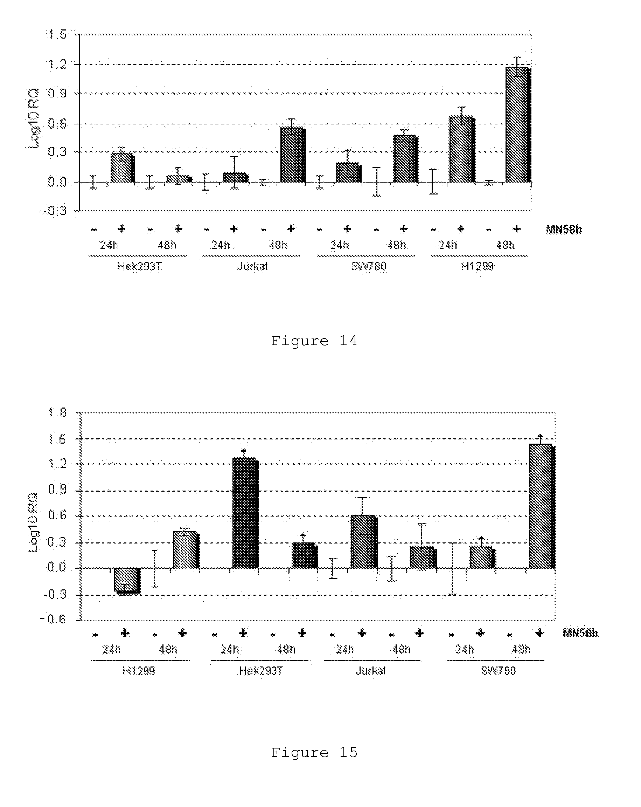

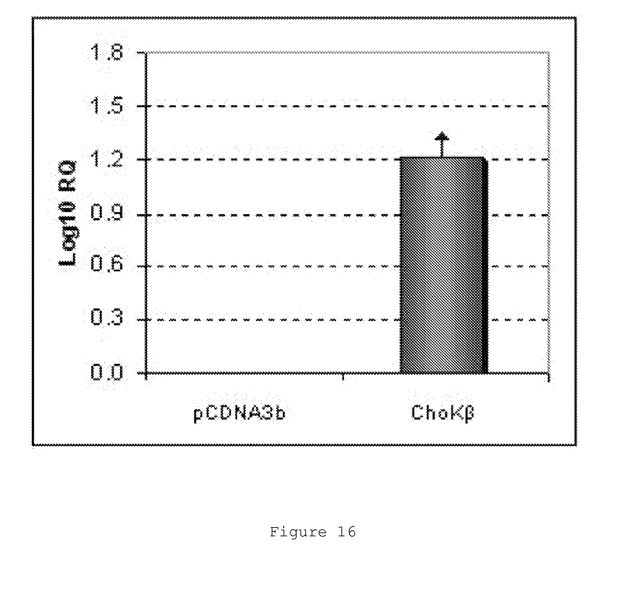

Figure 14. Increase of the transcription of PEMT in response to the overexpression of Chokβ. Hek293T cells were transfected with the expression vector of Chokβ or with the empty pCDNA3b vector as a control. The expression of PEMT, which enzyme is not expressed in normal conditions in this system, as is observed in the control, is induced as a transcriptional response to the overexpression of Chokβ. The relative quantity of the mRNA of PEMT in Log₁₀RQ obtained by the 2^{-ΔΔCt} method is shown in the figure. The arrow indicates that since the expression control of said gene is not shown, the comparison is extrapolated to the maximum number of PCR cycles.

### Detailed Description of the Invention

### Method for determining the prognosis of a patient suffering from cancer based on the use of ChoKβ expression levels or on the relationship between ChoKα and ChoKβ expression levels.

The authors of the present invention have identified that ChoKβ expression levels are correlated with the survival of patients with cancer. In particular, reduced levels of ChoKβ determined in a tumor sample of a patient are indicative of the patient showing a poor prognosis. The use of ChoKβ as a biomarker to predict the prognosis of a subject who suffers from cancer is thus possible.

Thus, in one aspect, the invention relates to a method for determining the prognosis of a patient suffering from cancer (hereinafter, first prognosis method of the invention) comprising determining ChoKβ expression levels in a sample of said patient in which reduced levels of ChoKβ in relation to the levels in a reference sample are indicative of the patient showing a poor prognosis.

In addition, the authors of the present invention have demonstrated that the combined determination of the expression levels of two ChoK isoforms (ChoKβ and ChoKα) constitutes a prognostic factor with greater predictive value than the determination of each of them separately. Particularly, the authors of the present invention have observed that patients who simultaneously have high levels of ChoKα and reduced levels of ChoKβ show a worse prognosis characterized as survival or relapse frequency.

Thus, in another aspect, the invention relates to a method for determining the prognosis of a patient suffering from cancer (hereinafter, second prognosis method of the invention) comprising determining ChoKα and ChoKβ expression levels in a sample of said patient in which reduced levels of ChoKα and high levels of ChoKβ in relation to the expression levels of said proteins in a reference sample are indicative of the patient showing a good prognosis.

In the present invention "prognosis" is understood as the expected progression of a disease and relates to the assessment of the probability according to which a subject suffers from a disease as well as to the assessment of its onset, state of development, progression, or of its regression, and/or the prognosis of the course of the disease in the future. As will be understood by persons skilled in the art, such assessment normally may not be correct for 100% of the subjects to be diagnosed, although it preferably is correct. The term, however, requires that a statistically significant part of the subjects can be identified as suffering from the disease or having a predisposition thereto. If a part is statistically significant it can be determined simply by the person skilled in the art using several well known statistical evaluation tools, for example, determination of confidence intervals, determination of p values, Student's t-test, Mann-Whitney test, etc. Details are provided in Dowdy and Wearden, Statistics for Research, John Wiley & Sons, New York 1983. The preferred confidence intervals are at least 50%, at least 60%, at least 70%, at least 80%, at least 90%, at least 95%. The p values are preferably 0.2, 0.1, 0.05.

The prediction of the clinical outcome can be done using any assessment criterion used in oncology and known by the person skilled in the art. The assessment parameters useful for describing the progression of a disease include:
■ disease-free progression which, as used herein, describes the ratio of subjects in complete recurrence who have not had disease relapse during the time period under study;
■ objective response, which, as used in the present invention, describes the ratio of people treated in whom a complete or partial response is observed;
■ tumor control, which, as used in the present invention, relates to the ratio of people treated in whom a complete response, partial response, minor response or stable disease ≥ 6 months is observed;
■ progression-free survival which, as used herein, is defined as the time from the beginning of the treatment until the first measurement of cancer growth.
■ progression-free survival of six months or "PFS6" rate which, as used herein, relates to the percentage of people who are progression-free in the first six months after the beginning of the therapy
■ median survival which, as used herein, relates to the time in which half of the patients enrolled in the study are still alive, and
■ progression time, as used herein, relates to the time after which the disease is diagnosed (or treated) until the disease worsens.

In a particular embodiment of the invention, the clinical outcome is measured as subject survival or relapse-free survival.

As used herein, the term "subject" relates to all the animals classified as mammals and includes but is not limited to domestic and farm animals, primates and humans, for example, human beings, non-human primates, cows, horses, pigs, sheep, goats, dogs, cats, or rodents. Preferably, the subject is a male or female human being of any age or race.

To perform the prognosis methods of the invention, a sample of the subject under study is obtained. As used herein, the term "sample" relates to any sample which can be obtained from the patient. The method present can be applied to any type of biological sample of a patient, such as a biopsy, tissue, cell or fluid (serum, saliva, semen, sputum, cerebrospinal fluid (CSF), tears, mucus, sweat, milk, brain extracts and the like) sample. In a particular embodiment, said sample is a tissue sample or a part of such tissue, preferably a tumor tissue sample or a part of such tumor tissue. Said sample can be obtained by means of conventional methods, for example, biopsy, using well known methods for the persons skilled in the related medical techniques. The methods for obtaining a sample of the biopsy include dividing a tumor into large pieces, or microdissection or other cell separation methods known in the art. The tumor cells can be additionally obtained by means of fine needle aspiration cytology. To simplify the storage and the handling of the samples, they can be fixed in formalin and embedded in paraffin or first frozen and then embedded in a cryosolidifiable medium, such as OCT compound, by means of immersion in a highly cryogenic medium that allows for fast freeze.

As understood by the person skilled in the art, ChoKβ and/or ChoKα expression levels can be determined by measuring the levels of the mRNA encoded by said genes or by measuring the levels of proteins encoded by said genes, i.e., ChoKβ or ChoKα protein.

Thus, in a particular embodiment of the invention, the ChoKβ and/or ChoKα expression levels are determined by measuring the expression levels of the mRNA encoded by the ChoKβ and/or ChoKα gene. For this purpose, the biological sample can be treated to physically or mechanically break down the structure of the tissue or cell to release the intracellular components in an aqueous or organic solution to prepare the nucleic acids for additional analyses. The nucleic acids are extracted from the sample by means of known processes for the person skilled in the art and commercially available. The RNA is then extracted from frozen or fresh samples by means of any of the typical methods in the art, for example Sambrook, J., et al., 2001 Molecular Cloning, A Laboratory Manual, 3rd ed., Cold Spring Harbor Laboratory Press, N.Y., Vol. 1-3. Care is preferably taken to prevent the RNA from degrading during the extraction process.

In a particular embodiment, the expression level can be determined using the mRNA obtained from a tissue sample fixed in formalin, embedded in paraffin. The mRNA can be isolated from a pathological sample on file or a biopsy sample which is first deparaffinized. An exemplary deparaffinization method involves washing the sample in paraffin with an organic solvent, such as xylene. The deparaffinized samples can be rehydrated with an aqueous solution of a lower alcohol. The suitable lower alcohols include, for example, methanol, ethanol, propanols, and butanols. The deparaffinized samples can be rehydrated with successive washings with lower alcohol solutions with decreasing concentrations, for example. Alternatively, the sample is deparaffinized and rehydrated simultaneously. The sample is then lysed and the RNA is extracted from the sample.

While all the techniques for determining the gene expression profile (RT-PCR, SAGE, or TaqMan) are suitable for use when the previous aspects of the invention are performed, the mRNA expression levels are often determined by means of reverse transcription polymerase chain reaction (RT-PCR). In a particular embodiment, the mRNA expression levels of ChoKβ and/or ChoKα are determined by means of quantitative PCR, preferably real time PCR. The detection can be carried out in individual samples or in tissue microarrays.

It is possible to compare the mRNA expression levels of interest in the samples to be assayed with the expression of a control RNA to normalize the expression values of the mRNA among the different samples. As used herein, "control RNA" relates to an RNA the expression levels of which do not change or only change in limited amounts in tumor cells with respect to non-tumorigenic cells. The control RNA is preferably mRNA derived from maintenance genes and which encodes proteins which are constitutively expressed and which perform essential cell functions. Examples of maintenance genes for their use in the present invention include β-2-microglobulin, ubiquitin, 18-S ribosomal protein, cyclophilin, GAPDH and actin. In a preferred embodiment, the control RNA is β-actin mRNA. In one embodiment, the quantification of the relative gene expression is calculated according to the comparative Ct method using β-actin as endogenous control and commercial RNA controls as calibrators. The final results are determined according to the formula 2-(ΔCt of the sample-ΔCt of the calibrator), where the ΔCT values of the calibrator and the sample are determined by subtracting the target gene CT value from the β-actin gene value.

The determination of ChoKβ and/or ChoKα expression levels needs to be correlated with the reference values which correspond to the median value of the ChoKβ and/or ChoKα expression levels measured in a collection of tumor tissues in biopsy samples of subjects with cancer. Once this median value is established, the level of this marker expressed in tumor tissues of patients can be compared with this median value, and thus be assigned to the "low", "normal" or "high" expression level. The collection of samples from which the reference level is derived will preferably consist of subjects suffering from the same type of cancer.

Once this median value is established, the level of this marker expressed in tumor tissues of patients can be compared with this median value, and thus be assigned to the "increased" or "reduced" expression level. Due to the variability among subjects (for example, aspects concerning age, race, etc.), it is very difficult (if not virtually impossible) to establish absolute reference values of ChoKβ and/or ChoKα expression. Thus, in a particular embodiment, the reference values for "increased" or "reduced" expression of ChoKβ and/or ChoKα expression are determined by calculating the percentiles by conventional means which involves assaying a group of samples isolated from normal subjects (i.e., people without a cancer diagnosis) for ChoKβ and/or ChoKα expression levels. The "reduced" levels of ChoKβ can then preferably be assigned to samples in which ChoKβ expression levels are equal to or less than the 50^{th} percentile in the normal population, including, for example, expression levels equal to or less than the 60^{th} percentile in the normal population, equal to or less than the 70^{th} percentile in the normal population, equal to or less than the 80^{th} percentile in the normal population, equal to or less than the 90^{th} percentile in the normal population, and equal to or less than the 95^{th} percentile in the normal population. The "increased" ChoKα levels can then preferably be then assigned to samples in which the ChoKα expression levels are equal to or greater than the 50^{th} percentile in the normal population, including, for example, expression levels equal to or greater than the 60^{th} percentile in the normal population, equal to or greater than the 70^{th} percentile in the normal population, equal to or greater than the 80^{th} percentile in the normal population, equal to or greater than the 90^{th} percentile in the normal population, and equal to or greater than the 95^{th} percentile in the normal population.

Alternatively, in another particular embodiment, ChoKβ and/or ChoKα expression levels can be determined by measuring both the levels of the proteins encoded by said genes, i.e., ChoKβ and/or ChoKα protein, and the levels of variants thereof.

The determination of the expression levels of the proteins can be carried out by means of immunological techniques such as for example, ELISA, immunoblot or immunofluorescence. Immunoblot is based on the detection of proteins previously separated by means of gel electrophoresis in denaturing conditions and immobilized in a membrane, generally nitrocellulose, by means of incubation with a specific antibody and a development system (for example, chemoluminescence). Analysis by means of immunofluorescence requires the use of a specific antibody for the target protein for the analysis of the expression. ELISA is based on the use of antigens or antibodies labeled with enzymes so that the conjugates formed between the target antigen and the labeled antibody results in the formation of enzymatically active complexes. Given that one of the components (the antigen or the labeled antibody) are immobilized on a support, the antigen-antibody complexes are immobilized on the support and can thus be detected by means of adding a substrate which is converted by the enzyme into a product which is detectable by means of, for example, spectrophotometry or fluorometry.

When an immunological method is used, any antibody or reagent which is known to bind to the target proteins with high affinity can be used for detecting the amount of target proteins. However the use of an antibody, for example polyclonal sera, hybridoma supernatants or monoclonal antibodies, antibody fragments, Fv, Fab, Fab' and F (ab' ) 2, scFv, diabodies, triabodies, tetrabodies and humanized antibodies, is preferred.

In addition, the determination of the protein expression levels can be carried out by constructing a tissue microarray (TMA) containing the assembled samples of the subjects, and determining the expression levels of the proteins by means of immunohistochemical techniques well known in the state of the art.

Although the predictive methods of the invention can generally apply for any tumor type, in a preferred embodiment, both the first and the second predictive method of the invention are applied to tumors characterized by having high ChoKα expression levels.

The definition of high levels of ChoKα, the way to determine the levels and the reference sample suitable for determining said levels have been explained in detail in the context of the therapeutic method of the invention.

In a particular embodiment, the prognosis methods of the invention are applied to lung, breast, bladder or colorectal cancer.

### Method for determining the response of a patient with cancer to the treatment with a ChoKα inhibitor based on the use of the PEMT and/or ChoKβ levels

The authors of the present invention have surprisingly observed that there is a correlation between the PEMT and/or ChoKβ expression levels and the response of a patient with cancer to the treatment with ChoKα inhibitors. In particular, the results presented in Example 3 of the present invention indicate that high levels of ChoKβ and/or PEMT are correlated with a positive response to ChoKα inhibitors. These results allow the use of ChoKβ and/or of PEMT as biomarkers for response to ChoKα inhibitors. Thus, in another aspect, the invention relates to a method for determining the response of a patient with cancer to the treatment with a ChoKα inhibitor (hereinafter, method of personalized medicine of the invention) comprising determining in a sample of said patient the expression levels of a protein selected from the group of PEMT and ChoKβ, in which an increase of the PEMT expression levels or an increase of expression of the levels of ChoKβ in relation to the levels in a reference sample are indicative of a good response to the ChoKα inhibitor.

The expression "determining the response of a patient" relates to the assessment of the results of a therapy in a patient who suffers from cancer in response to a therapy based on the use of ChoKα inhibitors. The use of the biomarkers of the invention to monitor the efficacy of a treatment can also be applied to methods for selecting and screening drugs with potential anti-tumor activity. This process comprises a) administering the drug to be studied to the subject (preferably an animal); b) collecting biological samples of the animal at different points of the study (before, during and/or after the administration) and determining the marker levels according to the present invention; and c) comparing the determinations performed in the samples obtained in the different treatment phases and comparing them to control animals, for example untreated animals.

In the context of the present invention, PEMT is understood as the phosphatidylethanolamine methyltransferase protein, capable of catalyzing the conversion of phosphatidylethanolamine into phosphatidylcholine by means of double methylation.

As described in relation to the prognosis methods of the invention, the determination of the PEMT and ChoKβ levels can be carried out by means of the determination of the corresponding polypeptide levels, for which standard technology is used, such as Western-blot or immunoblot, E L I S A (enzyme-linked immunosorbent assay), RIA (radioimmunoassay), competitive EIA (enzyme immunoassay), DAS-ELISA (double antibody sandwich ELISA), immunocytochemical and immunohistochemical techniques, techniques based on the use of protein microarrays or biochips including specific antibodies or assays based on colloidal precipitation in formats such as reagent strips.

Alternatively, the determination of the PEMT and ChoKβ levels can be carried out by means of the determination of the corresponding mRNA levels, for which standard technology can be used, such as Real time PCR, SAGE, TaqMan, RT-PCR and the like.

In the case of PEMT, it is also possible to determine the expression levels by means of the determination of the enzyme activity of the corresponding protein, for which conventional methods are used, such as those based on the detection of the incorporation of methyl groups labeled with phosphatidyldimethylethanolamine using to that end [methyl-3H] AdoMet as the donor of methyl groups as originally described by Ridgway and Vance (Methods Enzymol. 1992, 209, 366-374), Zhu et al. (Biochem. J., 2003, 370, 987-993) and Song et al. (FASEB J., 2005, 19: 1266-1271).

In the context of the present invention, "ChoKα inhibitor" is understood as any compound capable of producing a decrease in the ChoK activity, including those compounds which prevent the expression of the ChoKα gene, causing reduced levels of mRNA or ChoK protein, as well as compounds which inhibit ChoK causing a decrease in the activity of the enzyme.

Compounds capable of preventing the expression of the ChoKα gene can be identified using standard assays for determining the mRNA expression levels such as RT-PCR, RNA protection analysis, Northern procedure, *in situ* hybridization, microarray technology and the like.

The compounds which cause reduced levels of ChoK protein can be identified using standard assays for determining the protein expression levels such as immunoblot or Western blot, ELISA (adsorption enzyme immunoanalysis), RIA (radioimmunoassay), competitive EIA (competitive enzyme immunoassay), DAS-ELISA (double antibody sandwich ELISA), immunocytochemical and immunohistochemical techniques, techniques based on the use of protein microarrays or biochip which include specific antibodies or assays based on colloidal precipitation in formats such as reagent strips.

The determination of the inhibiting capacity on the biological activity of choline kinase is detected using standard assays to measure the activity of choline kinase, such as methods based on the detection of the phosphorylation of choline labeled with [¹⁴C] by ATP in the presence of purified recombinant choline kinase or a choline kinase-rich fraction followed by detection of the phosphorylated choline using standard analytical techniques (for example, TLC) as described in EP1710236.

Exemplary choline kinase inhibitors that can be used in the first composition of the present invention are described in Table 1 from I to XVIII.

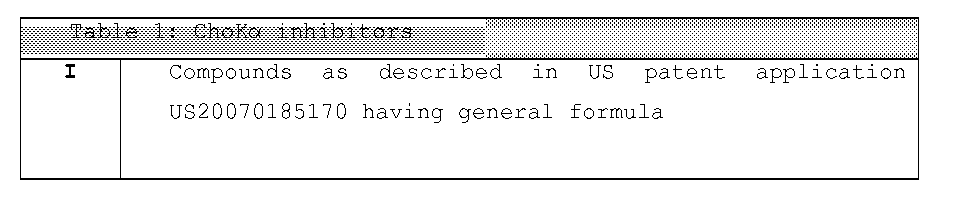

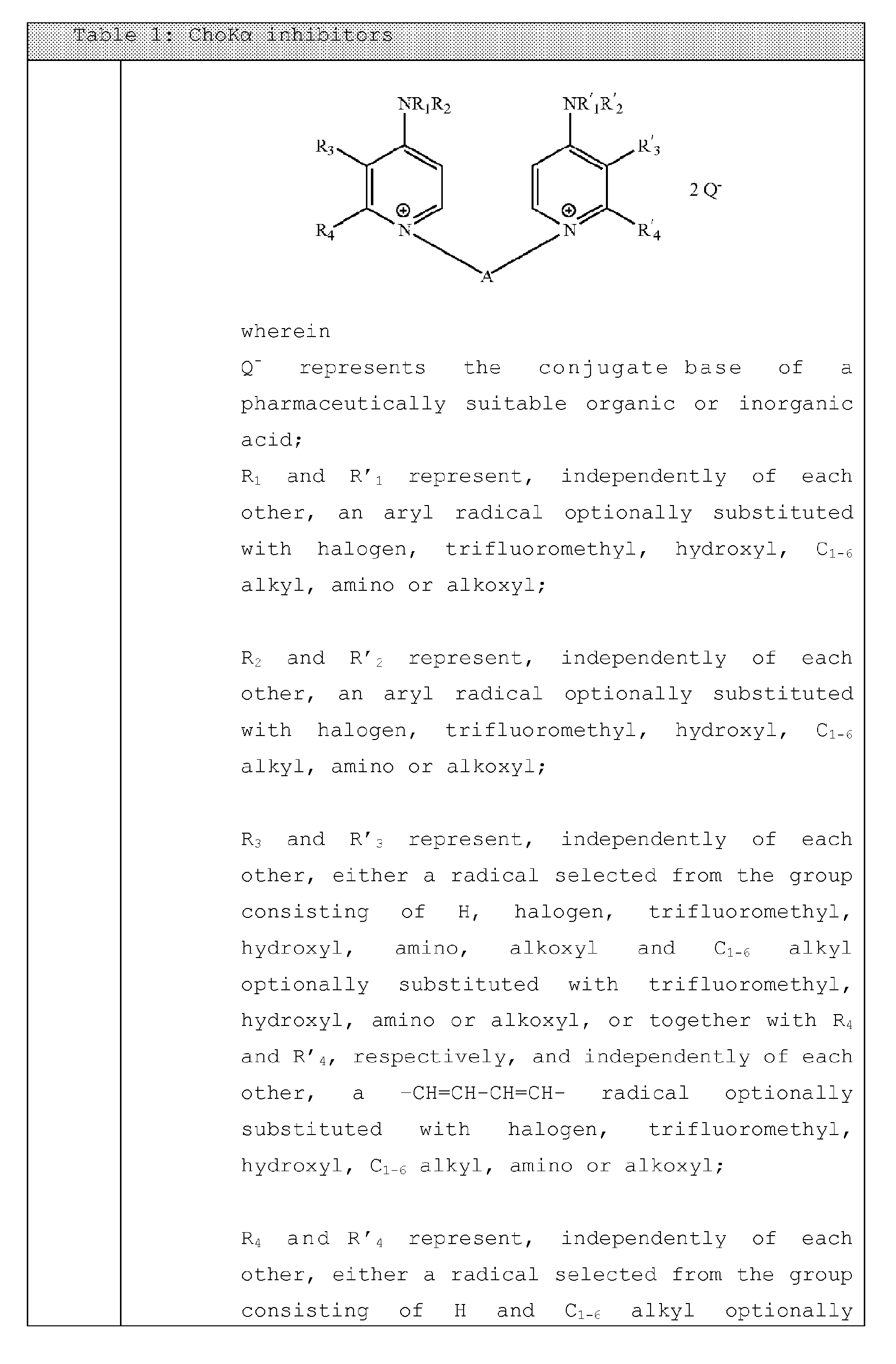

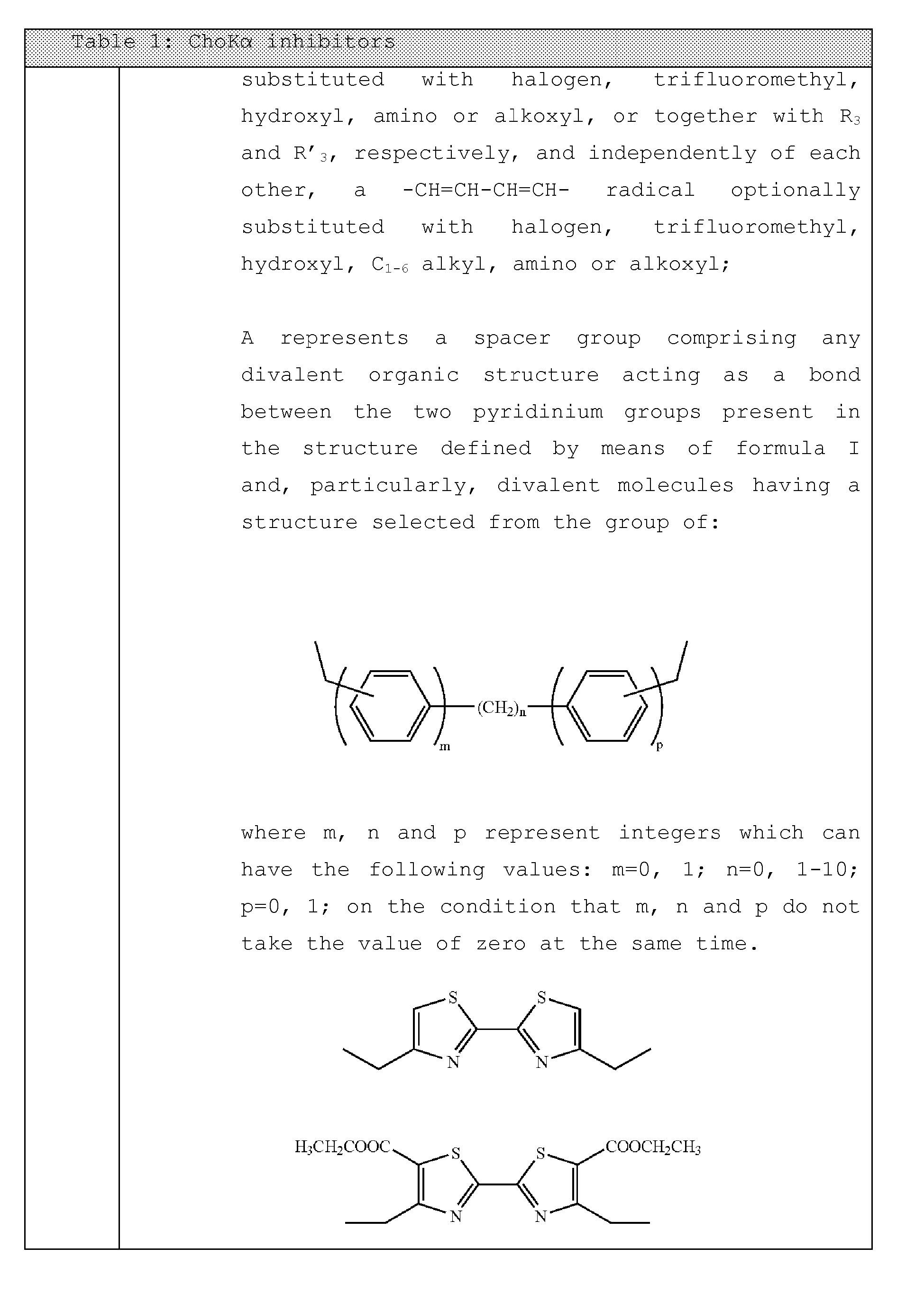

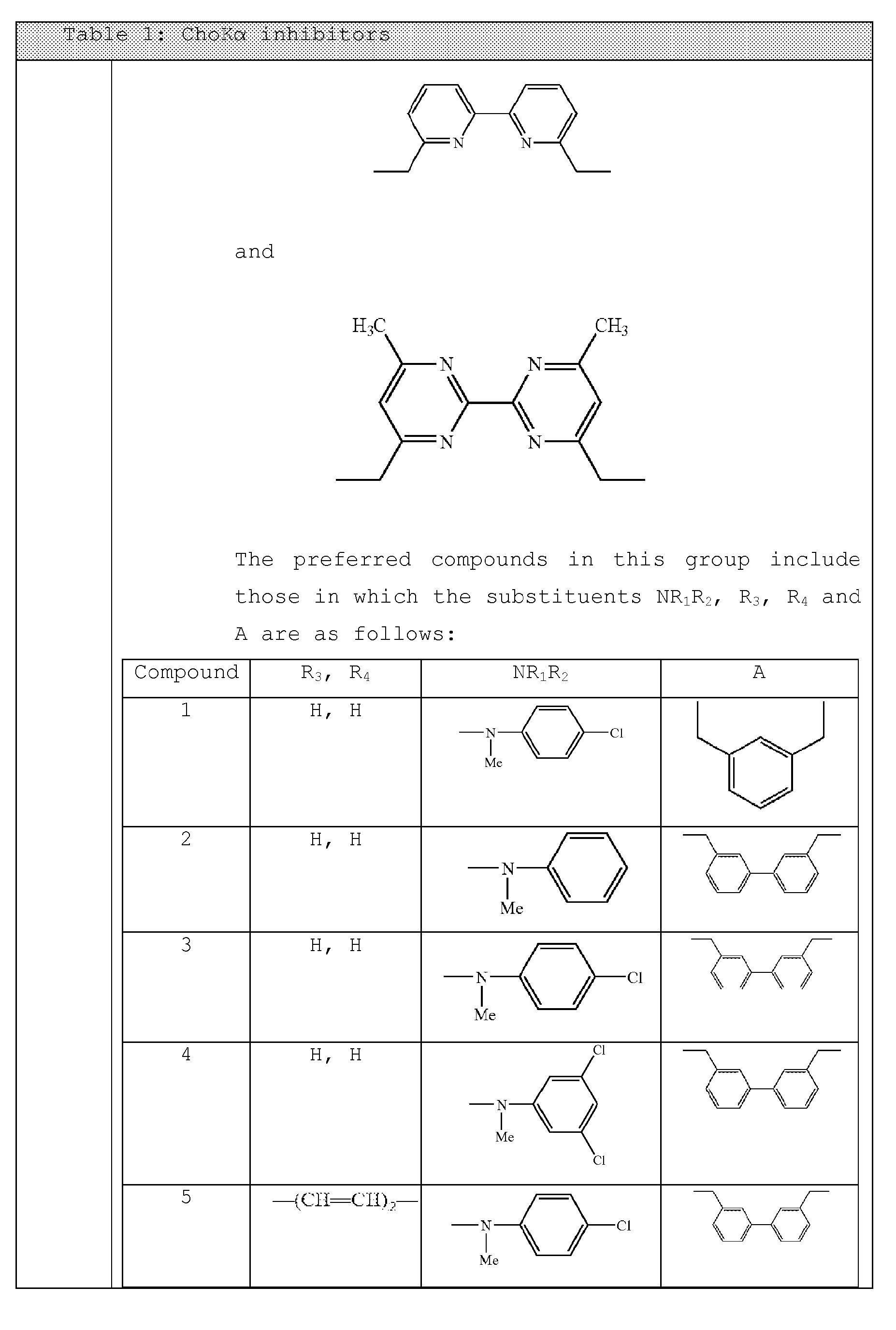

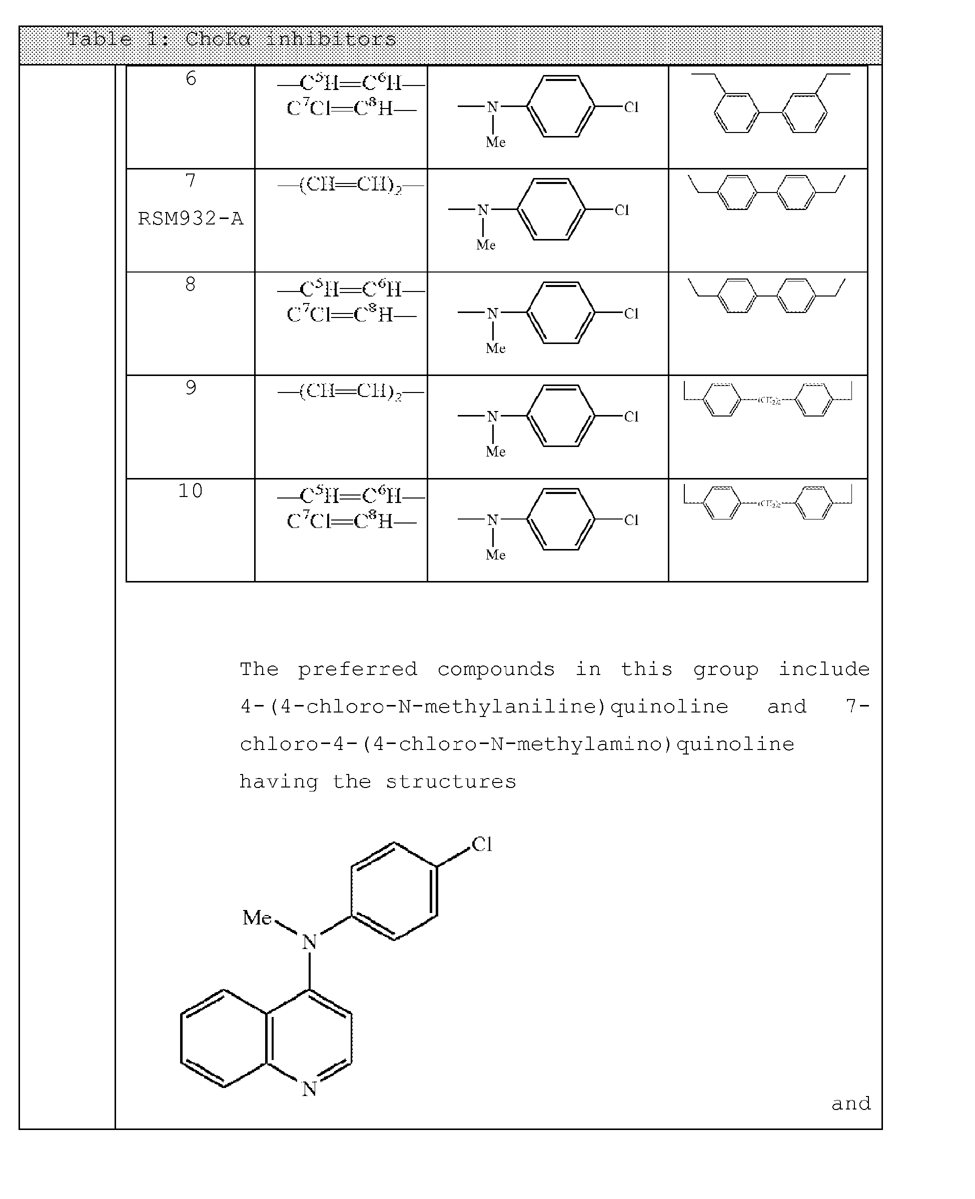

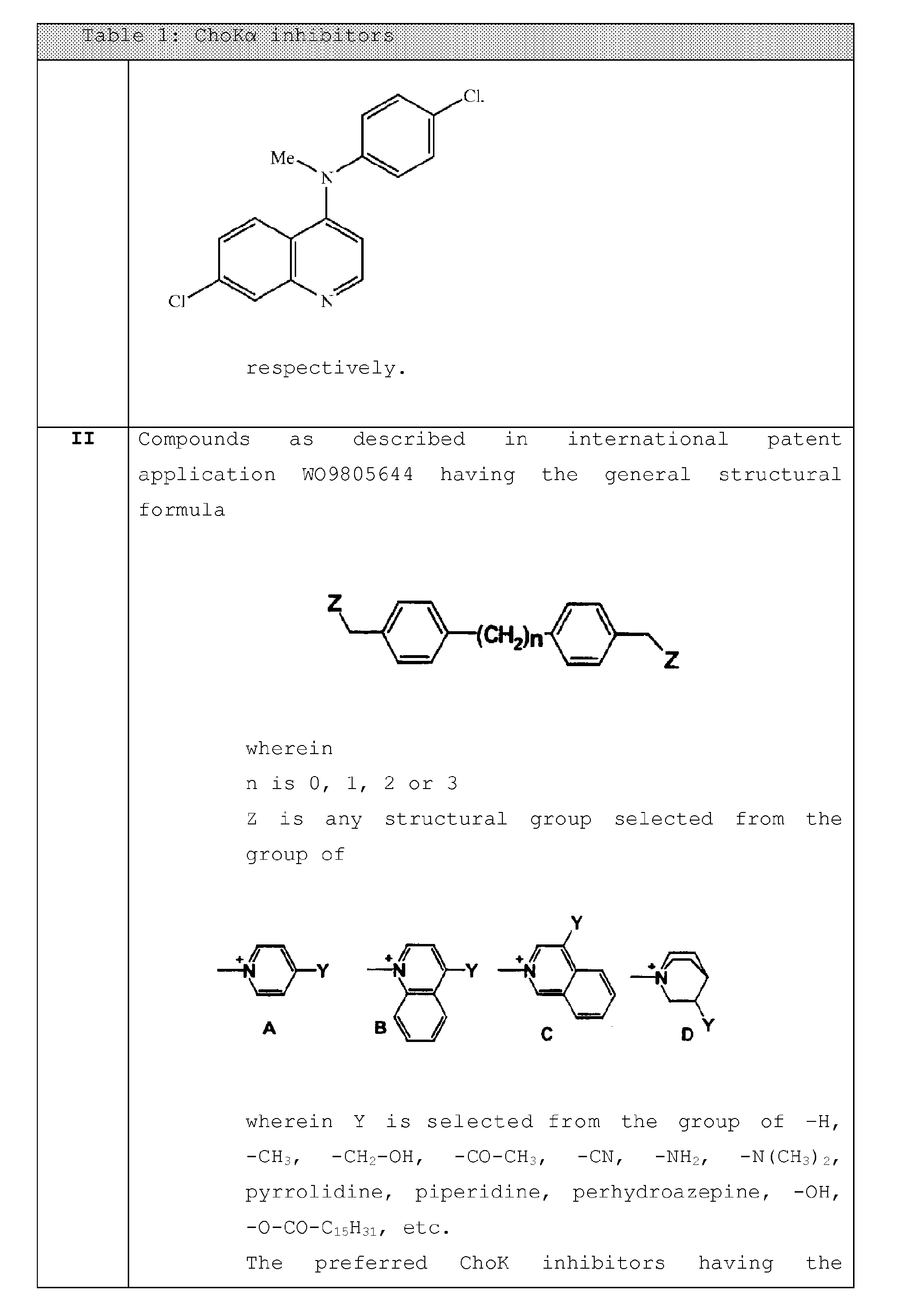

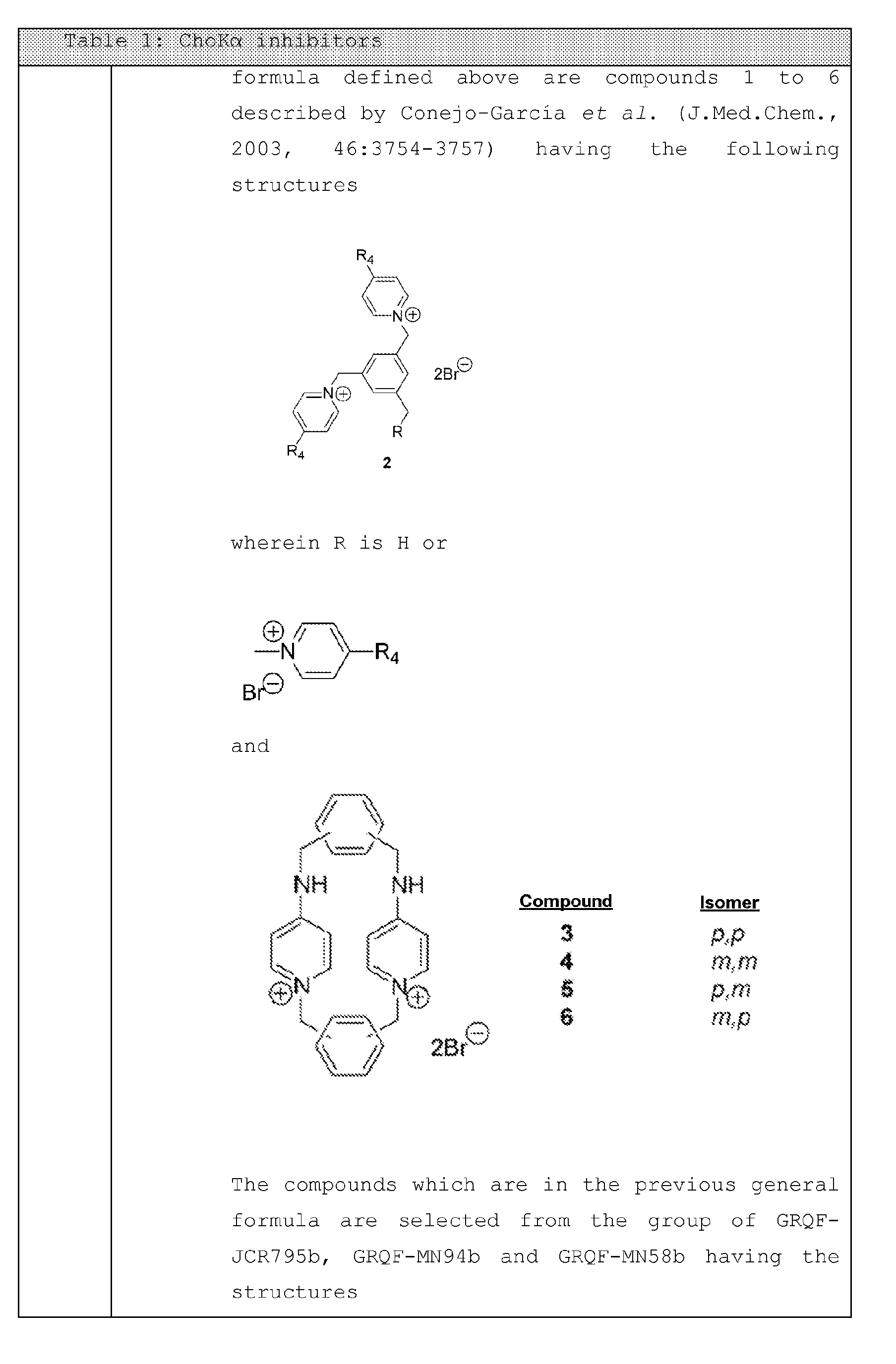

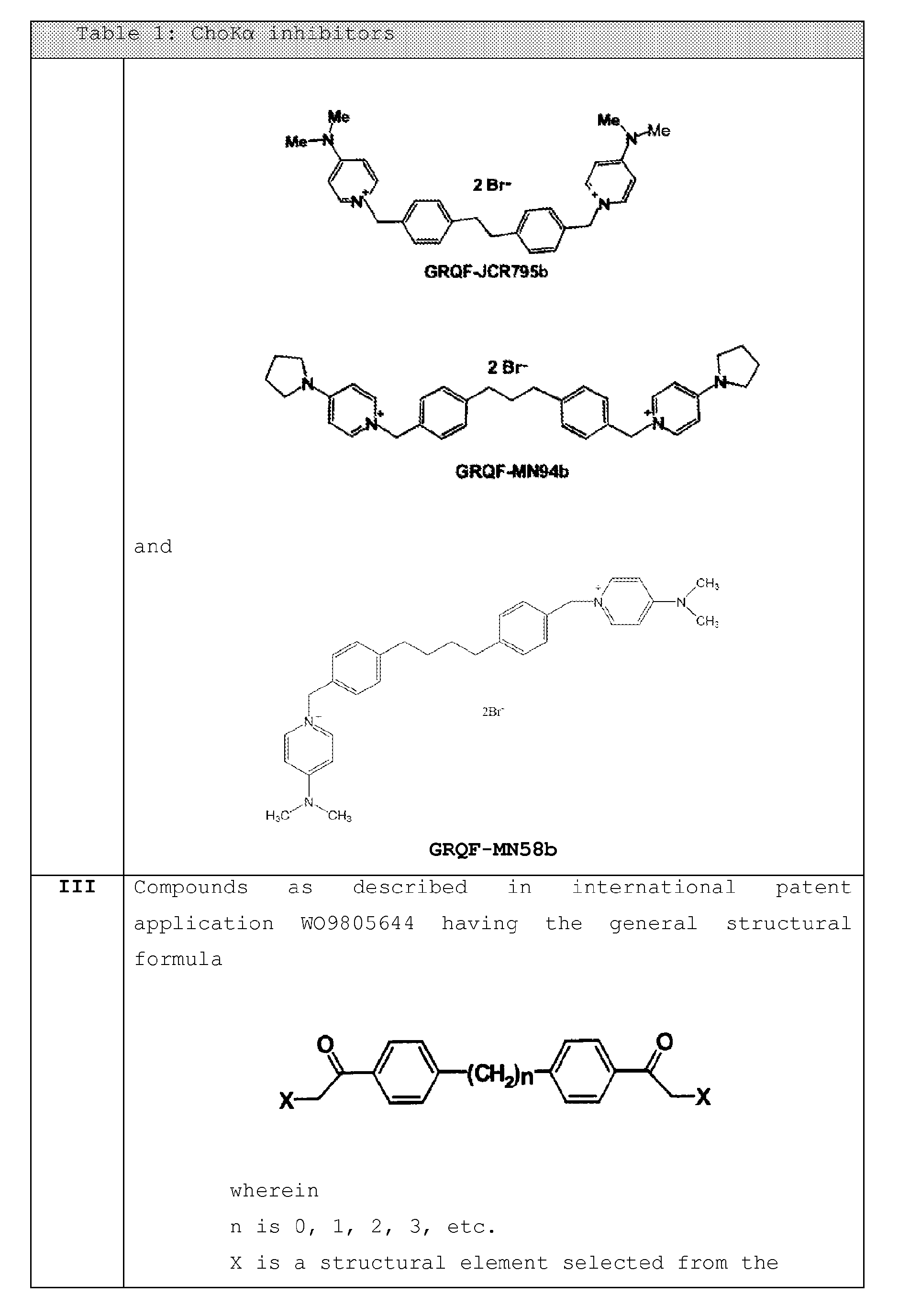

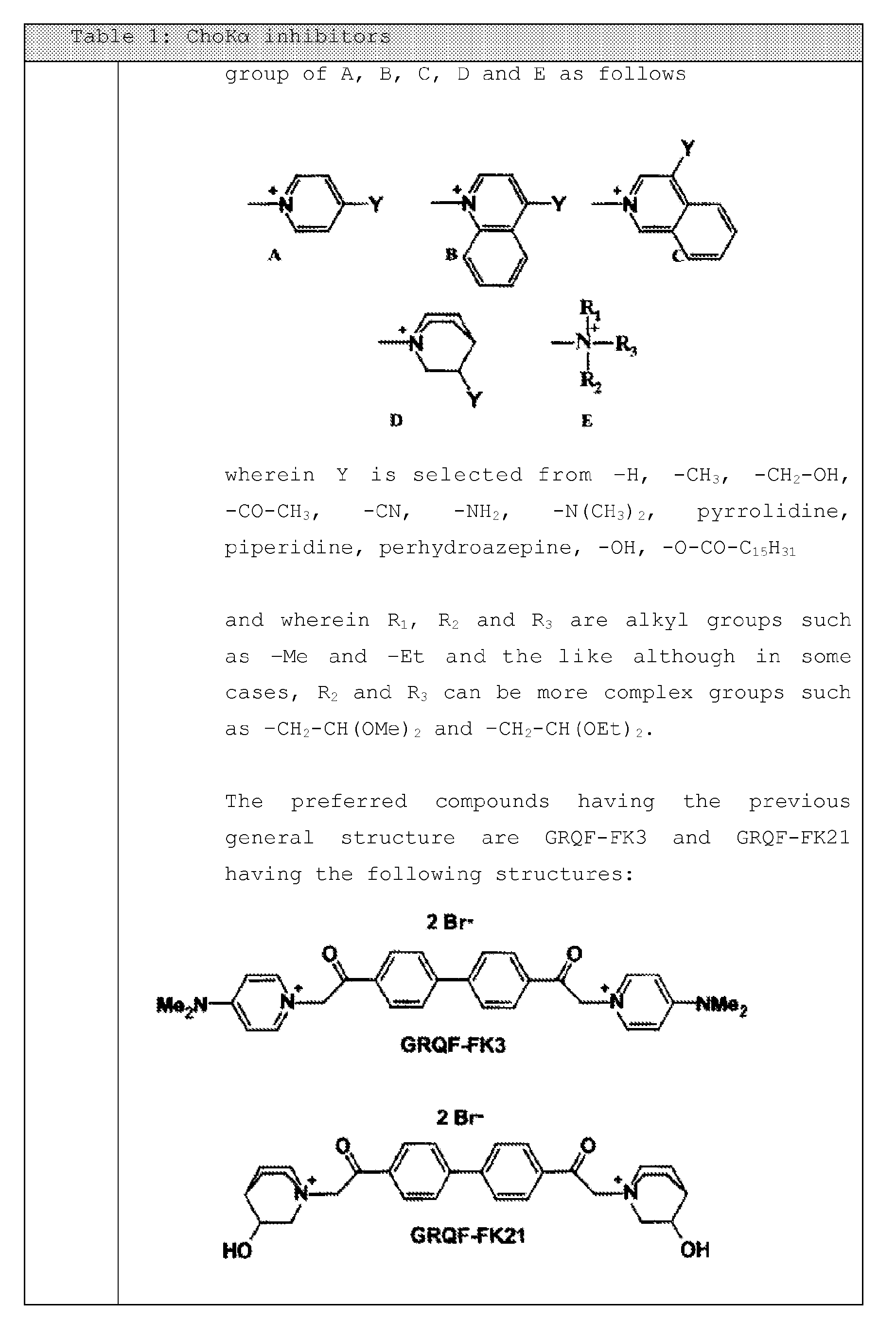

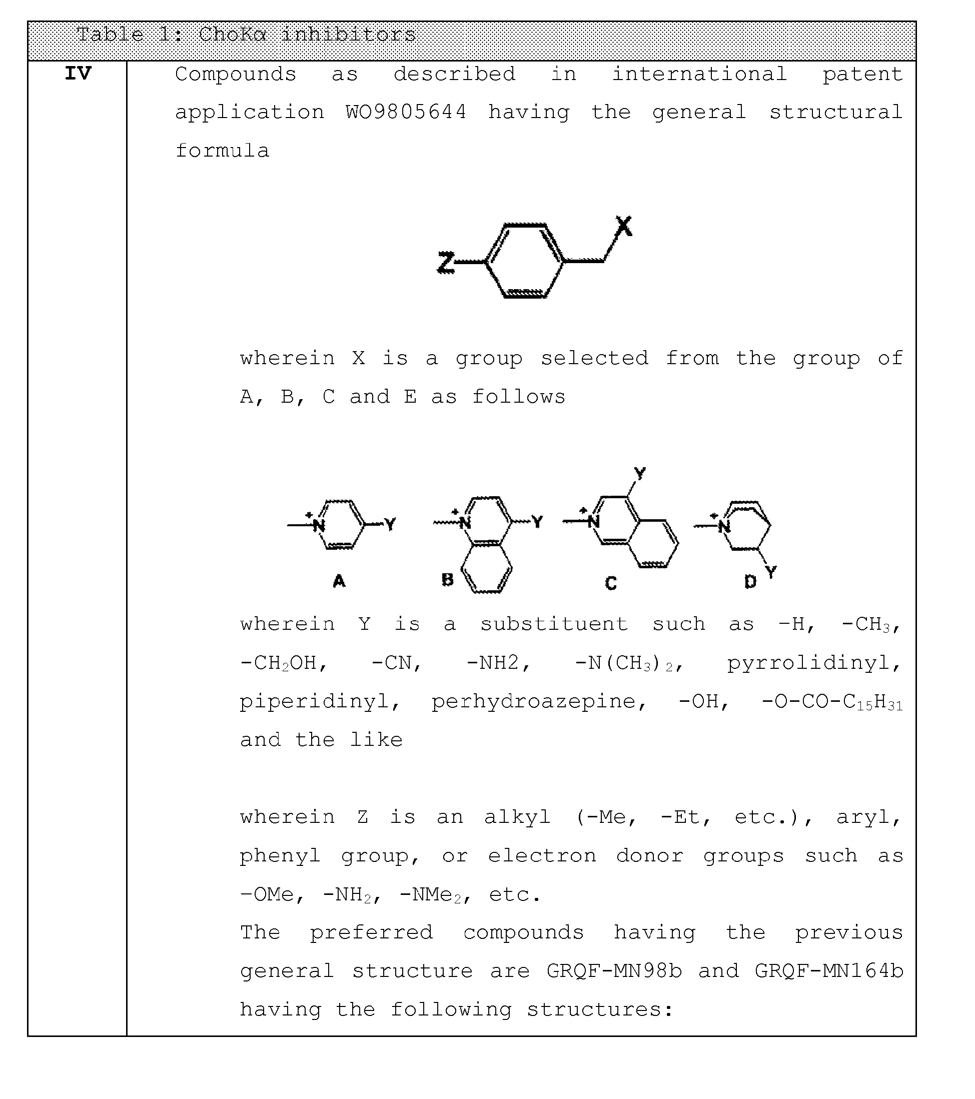

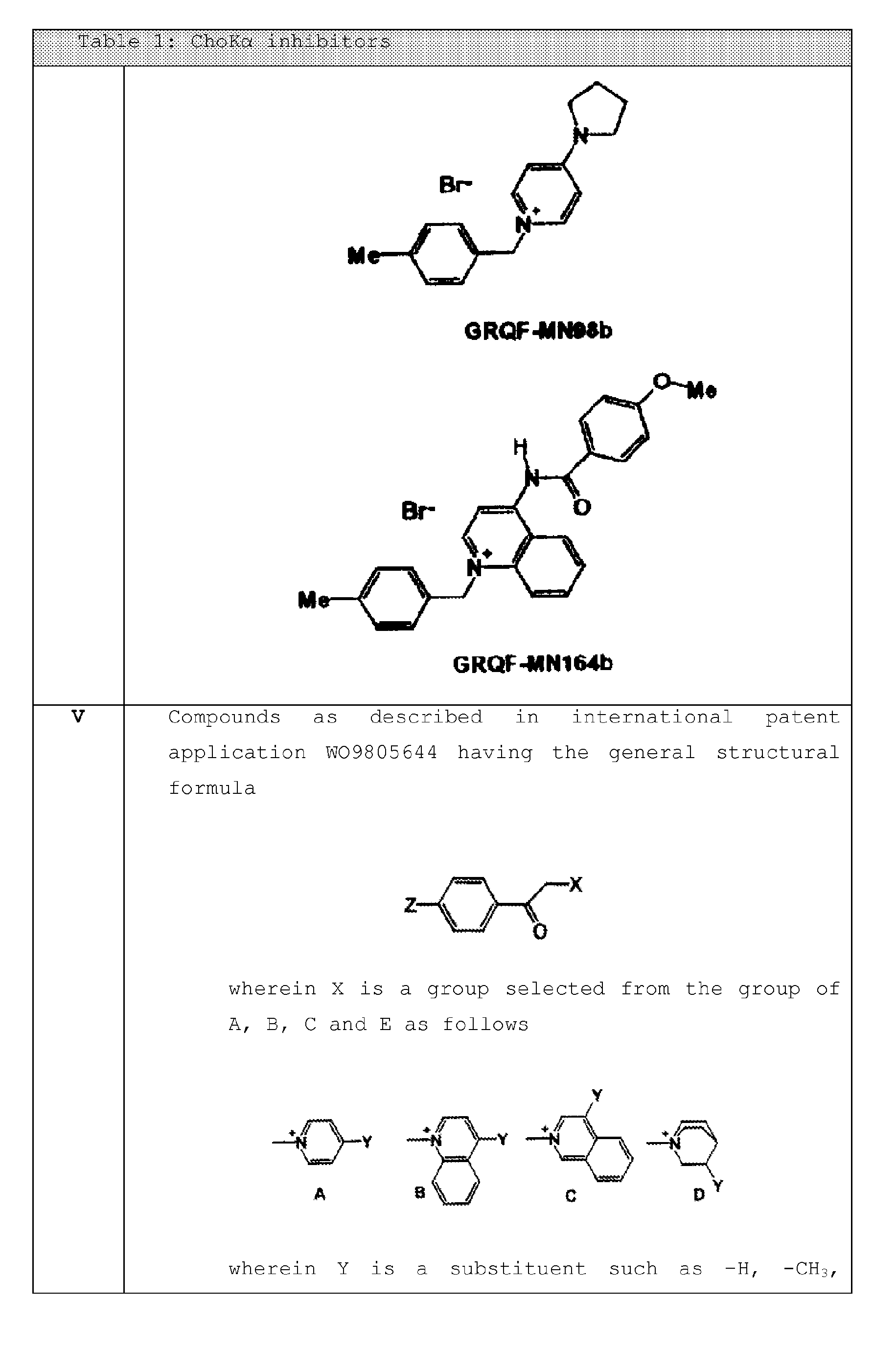

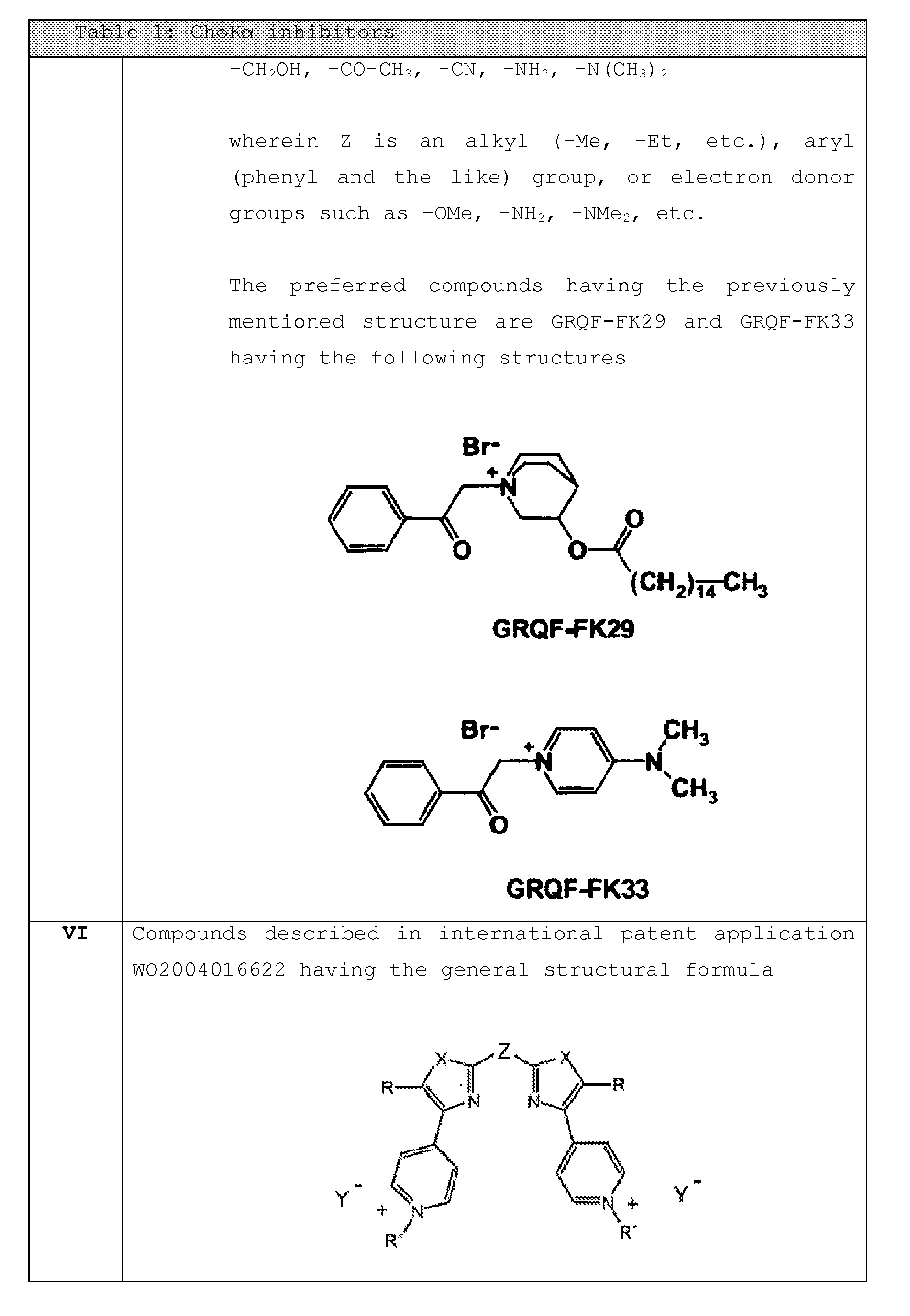

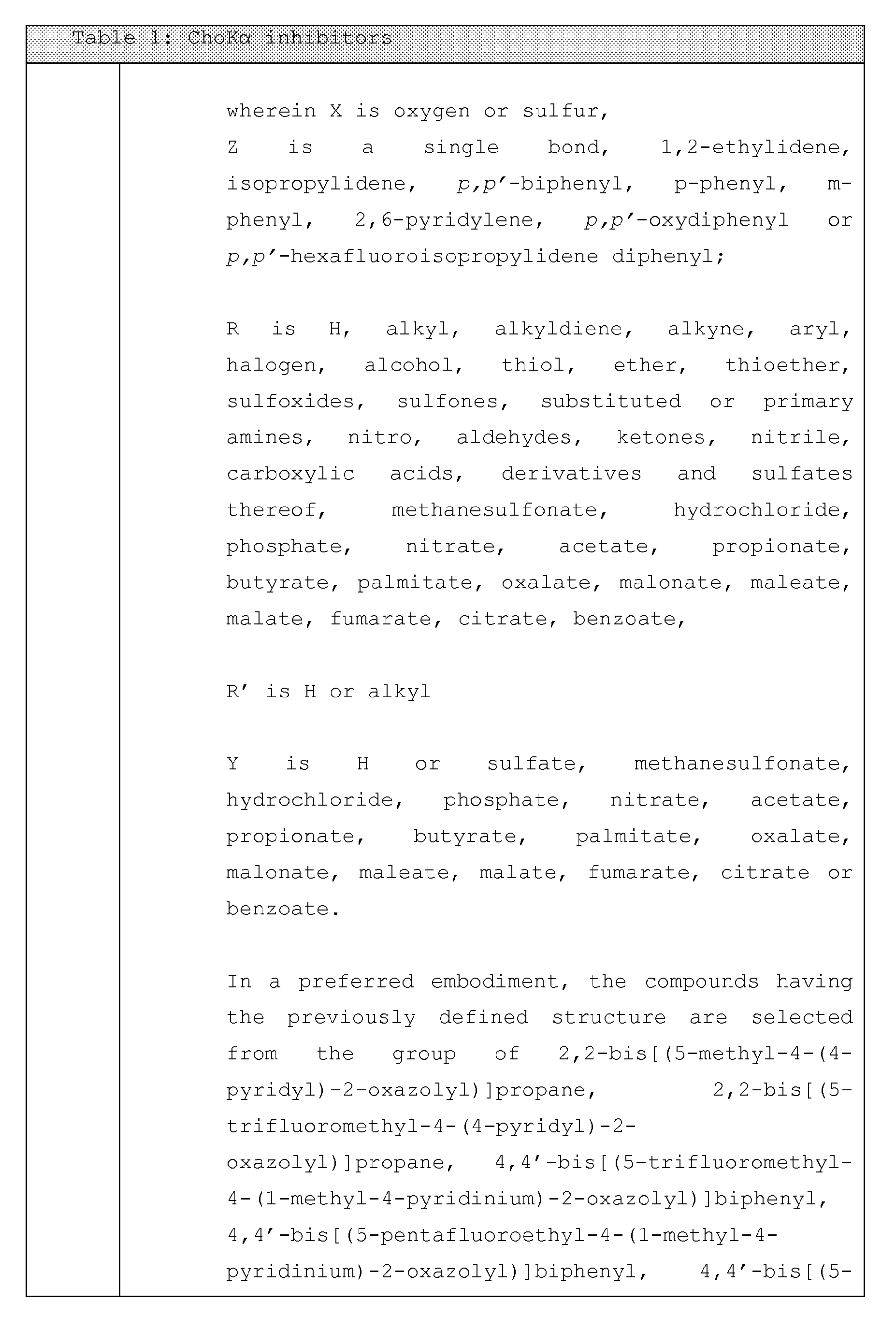

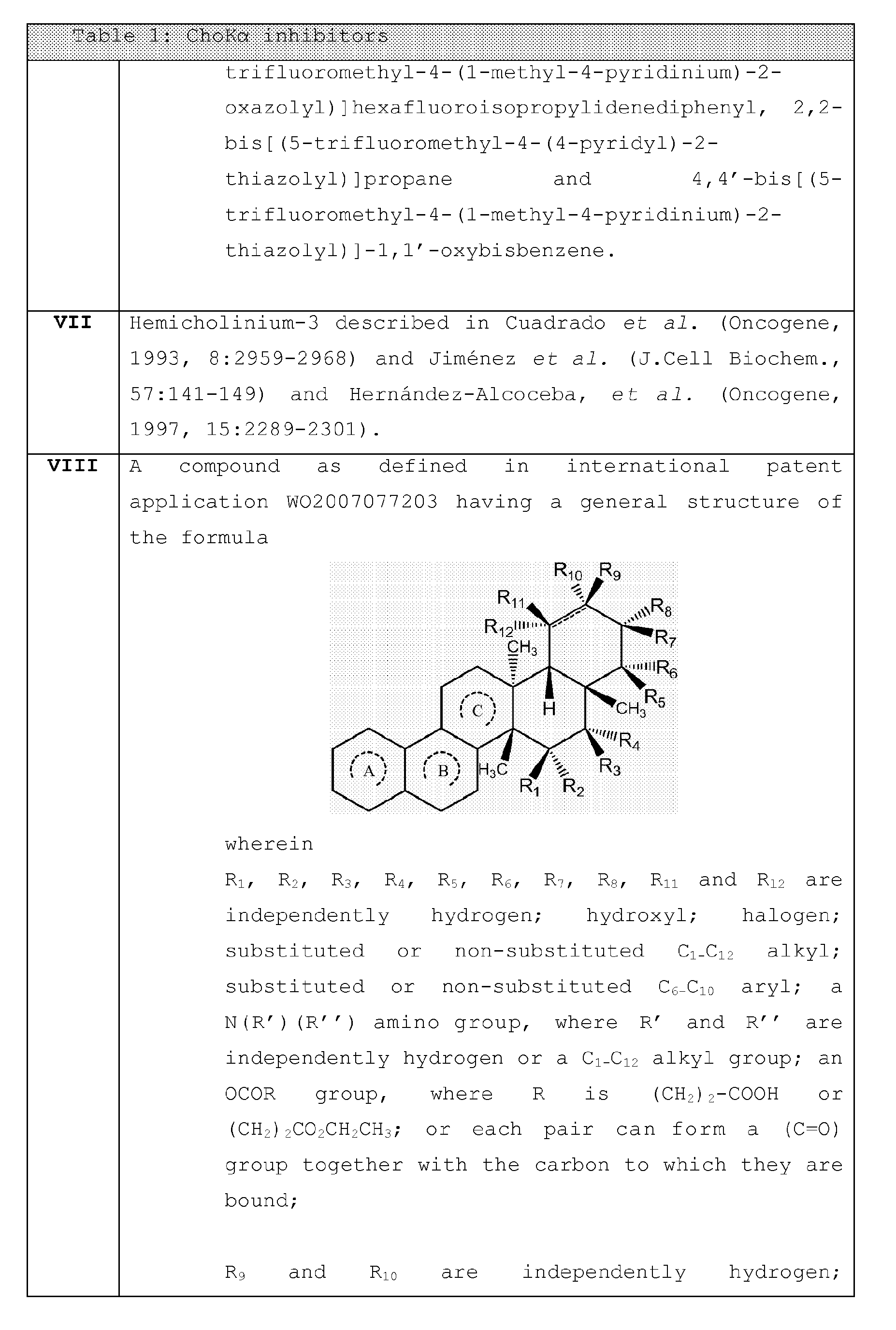

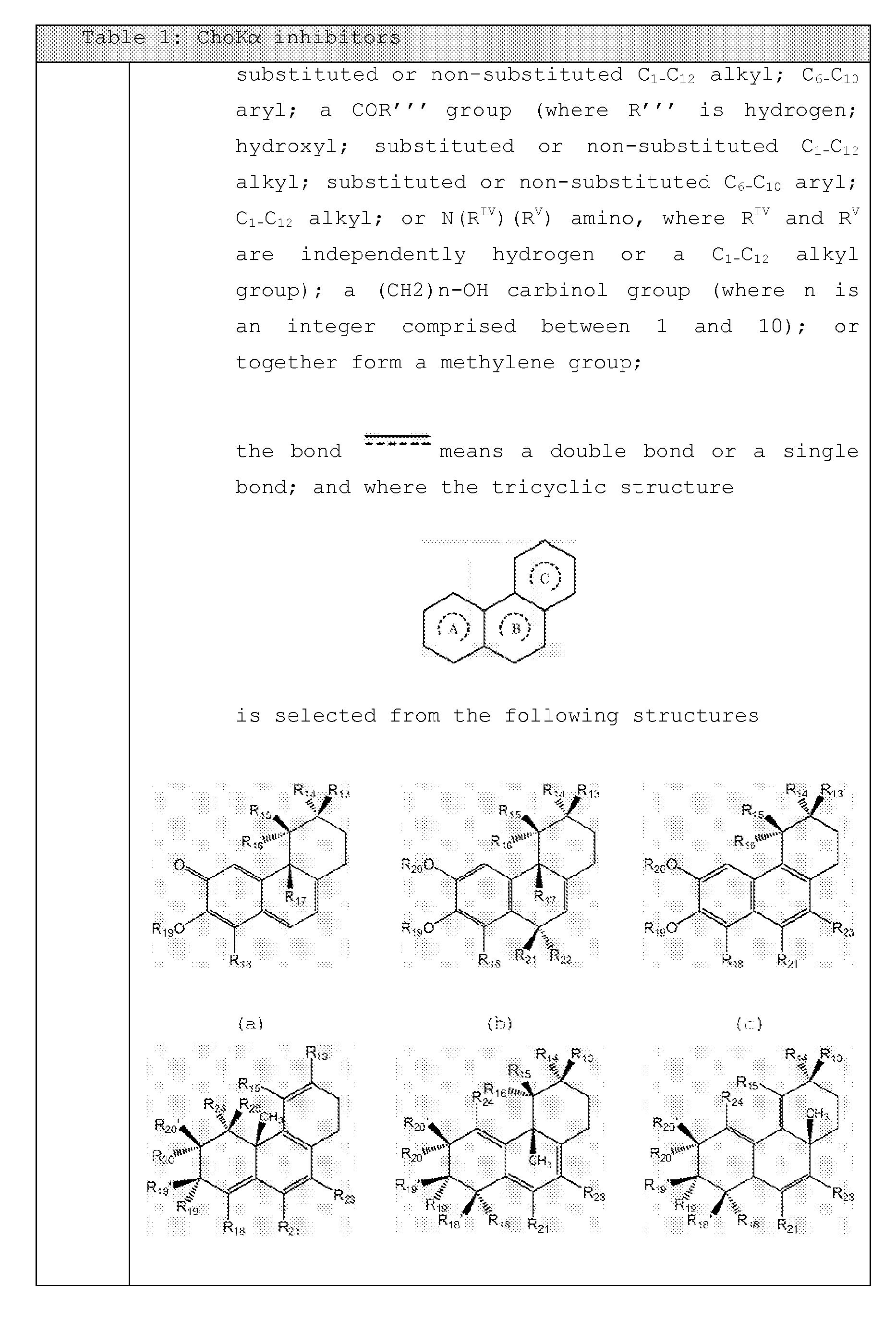

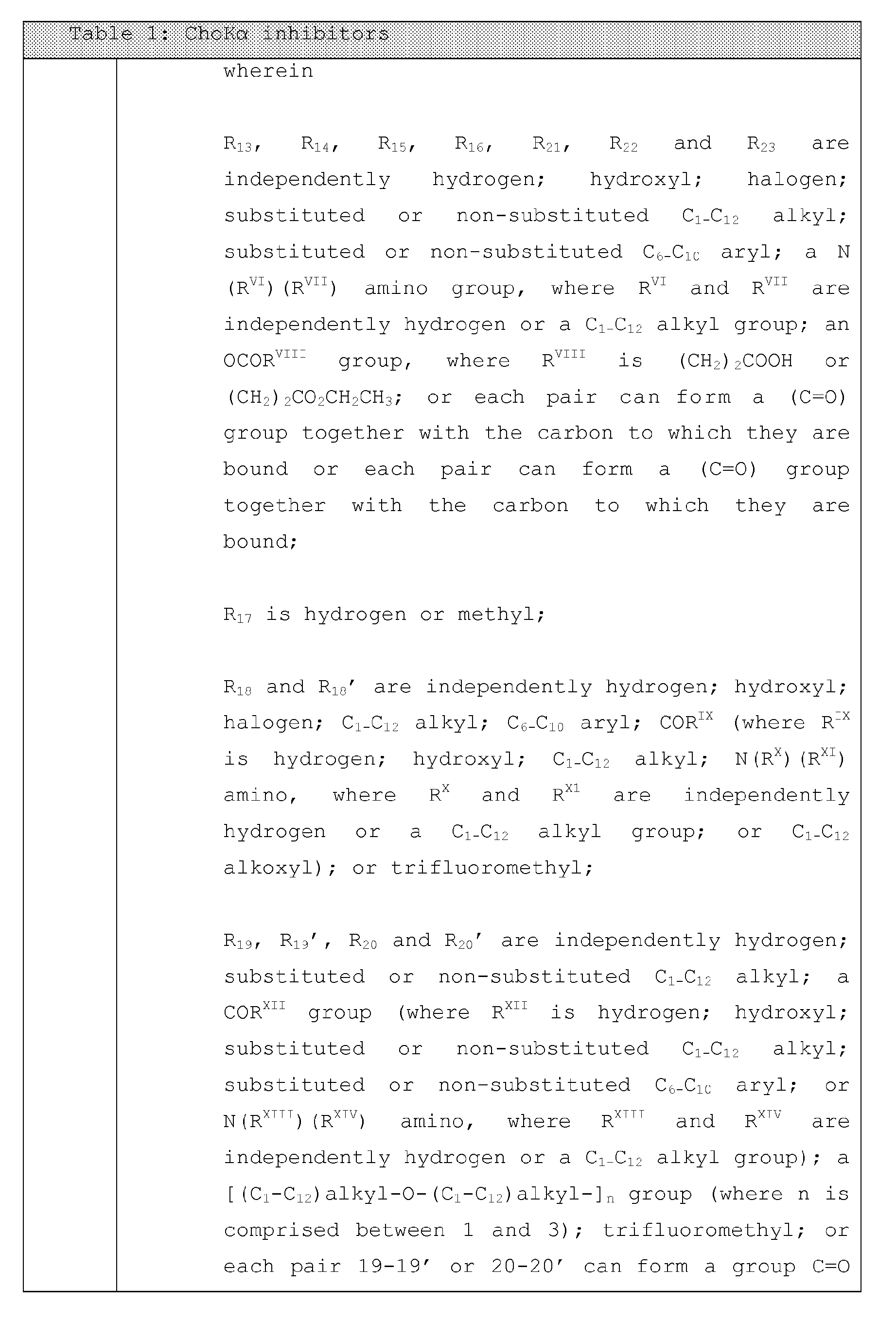

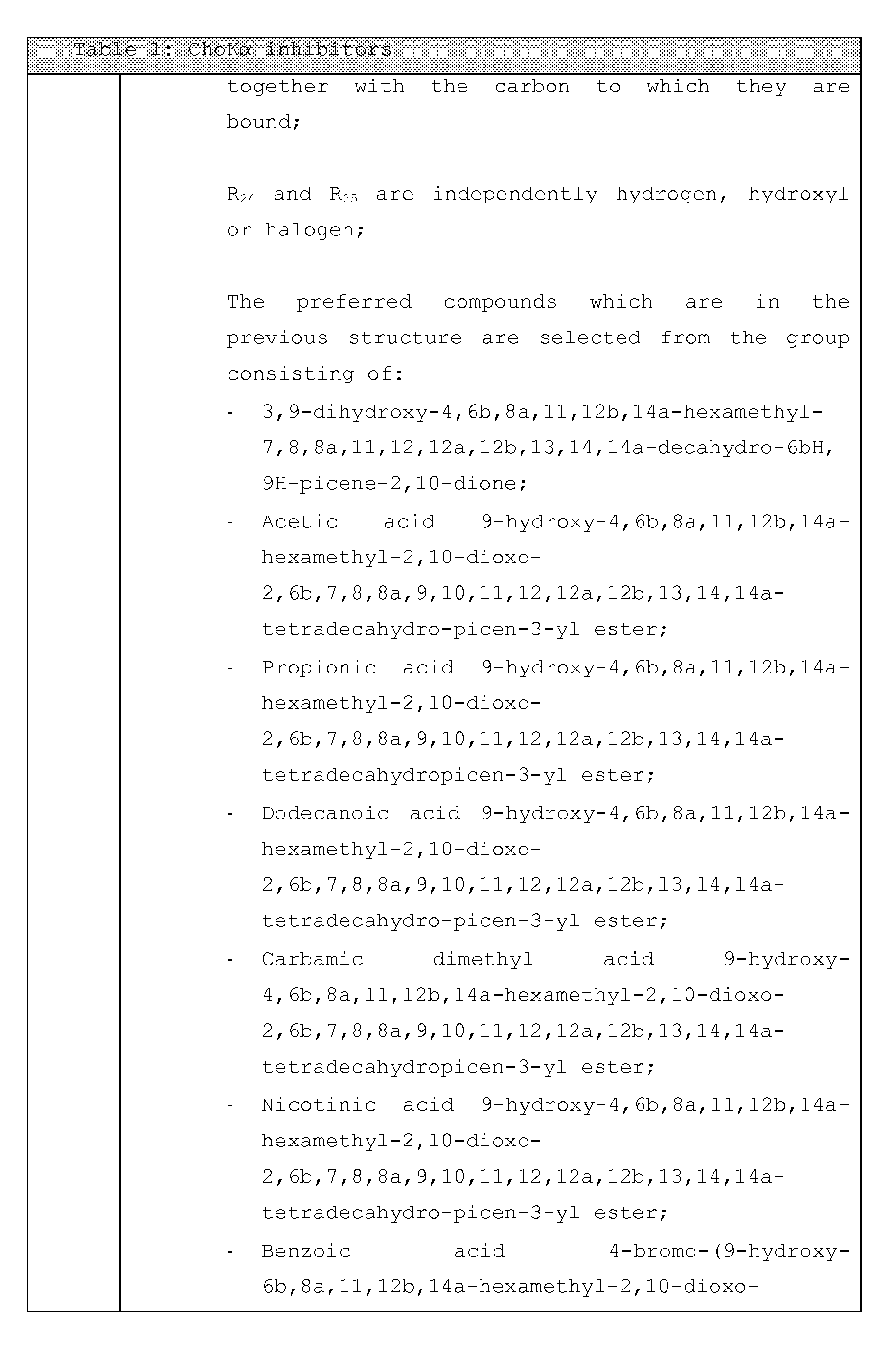

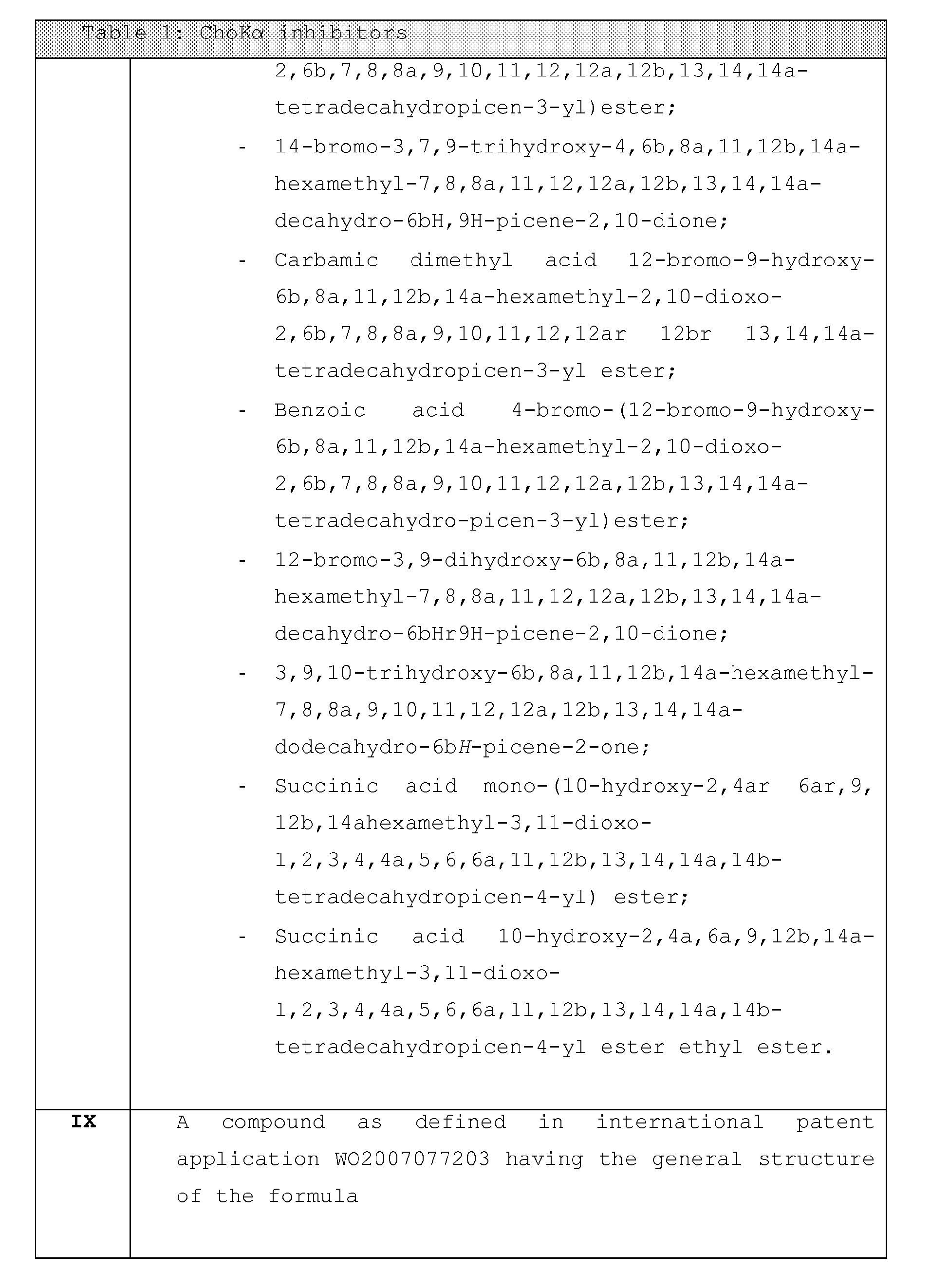

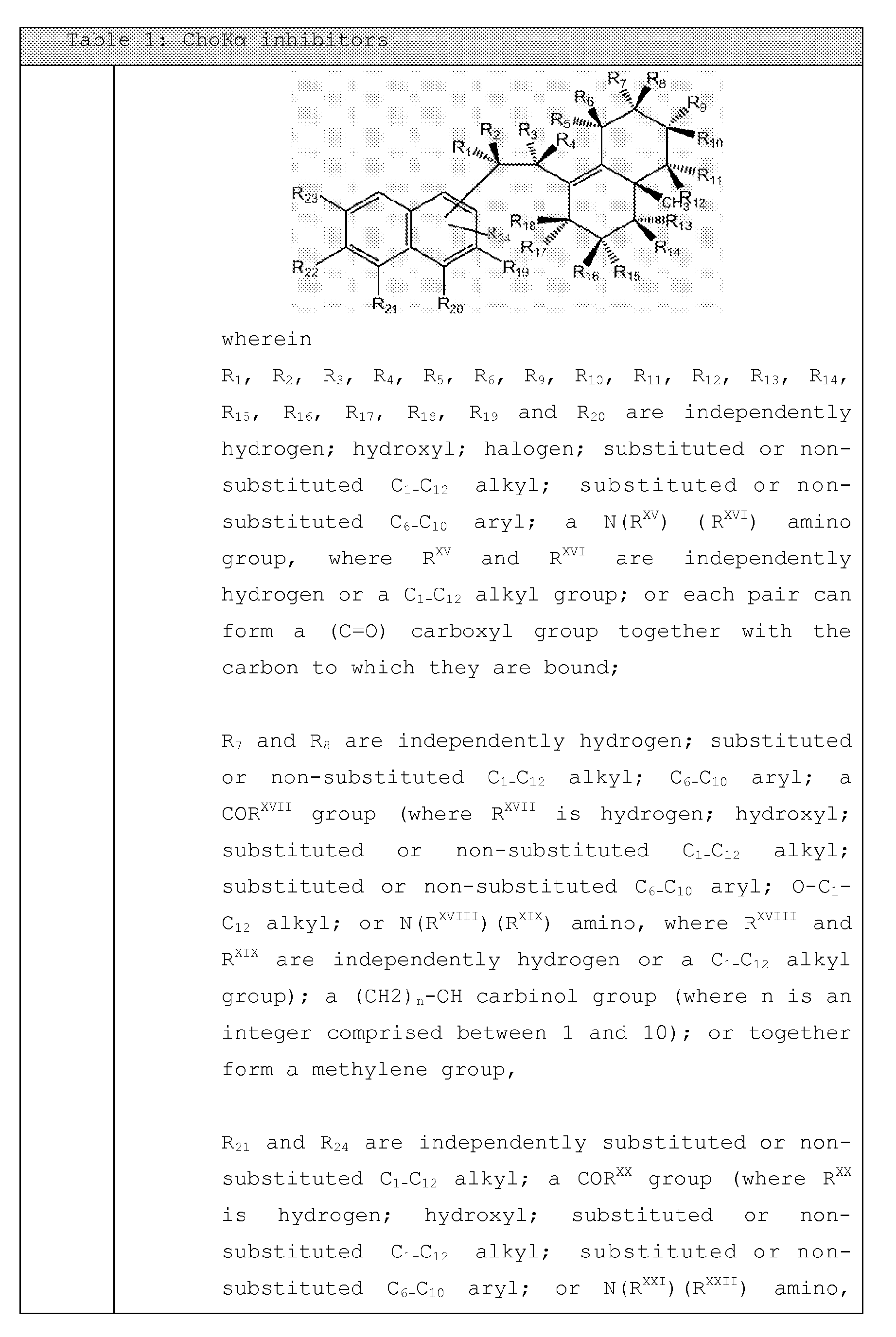

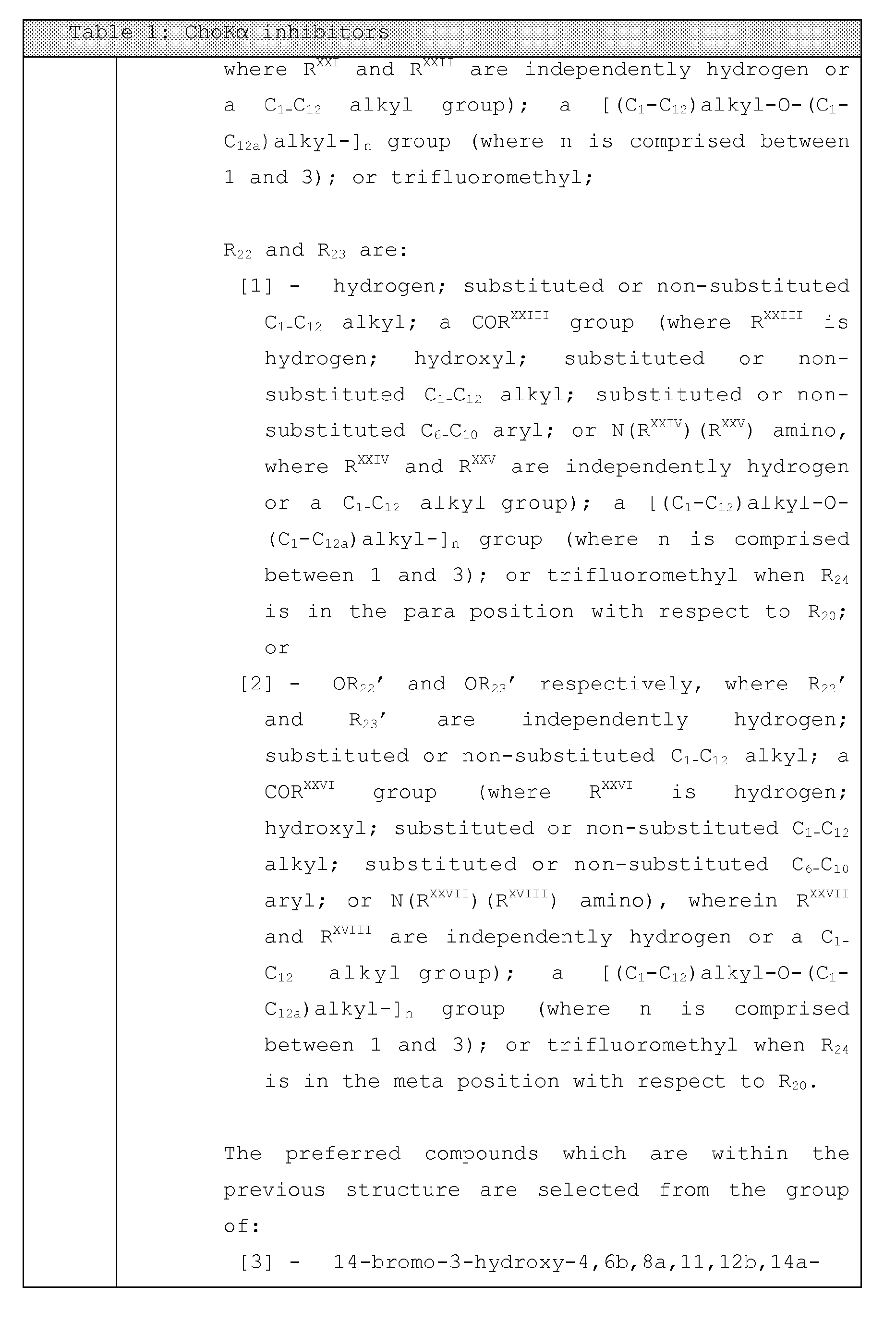

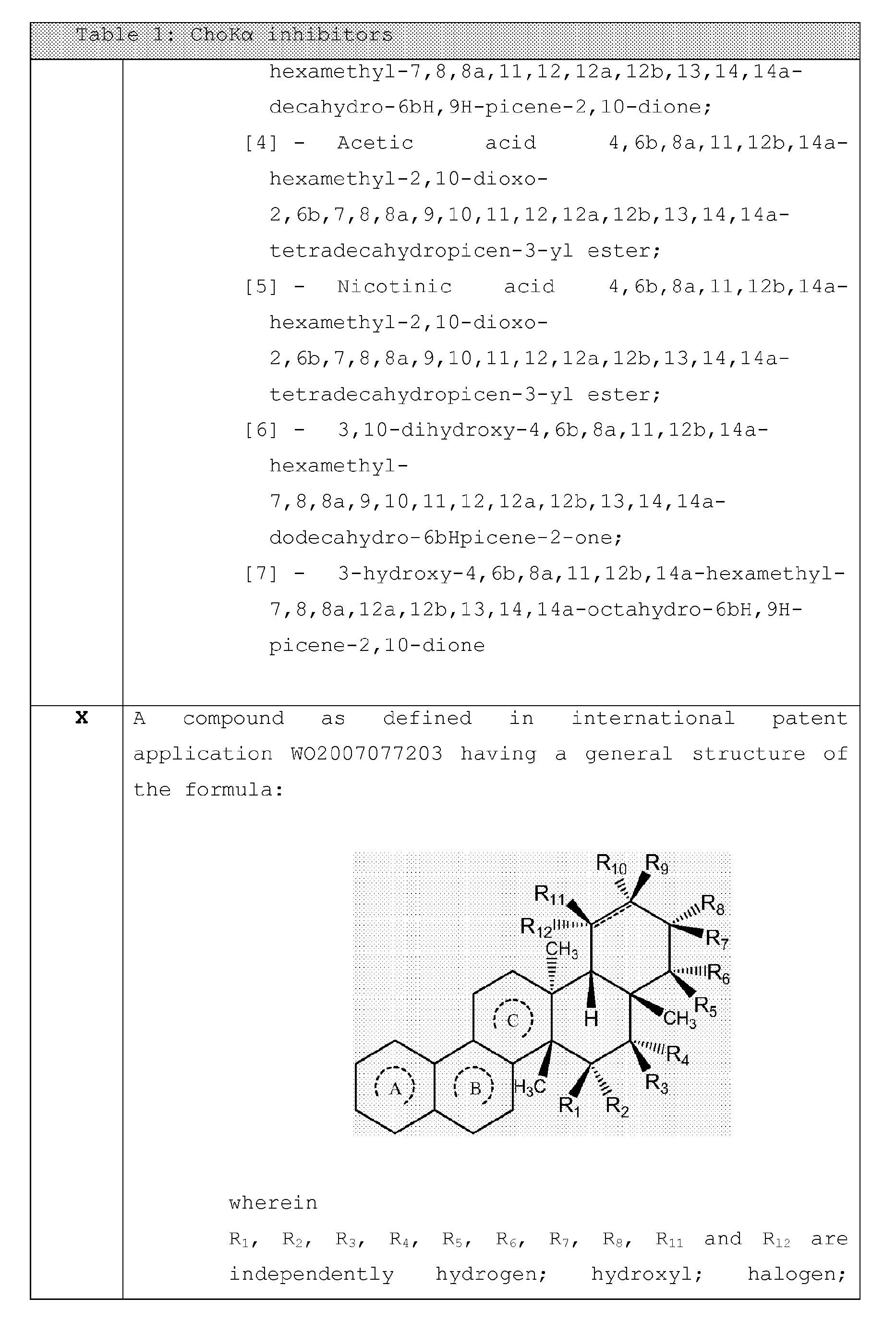

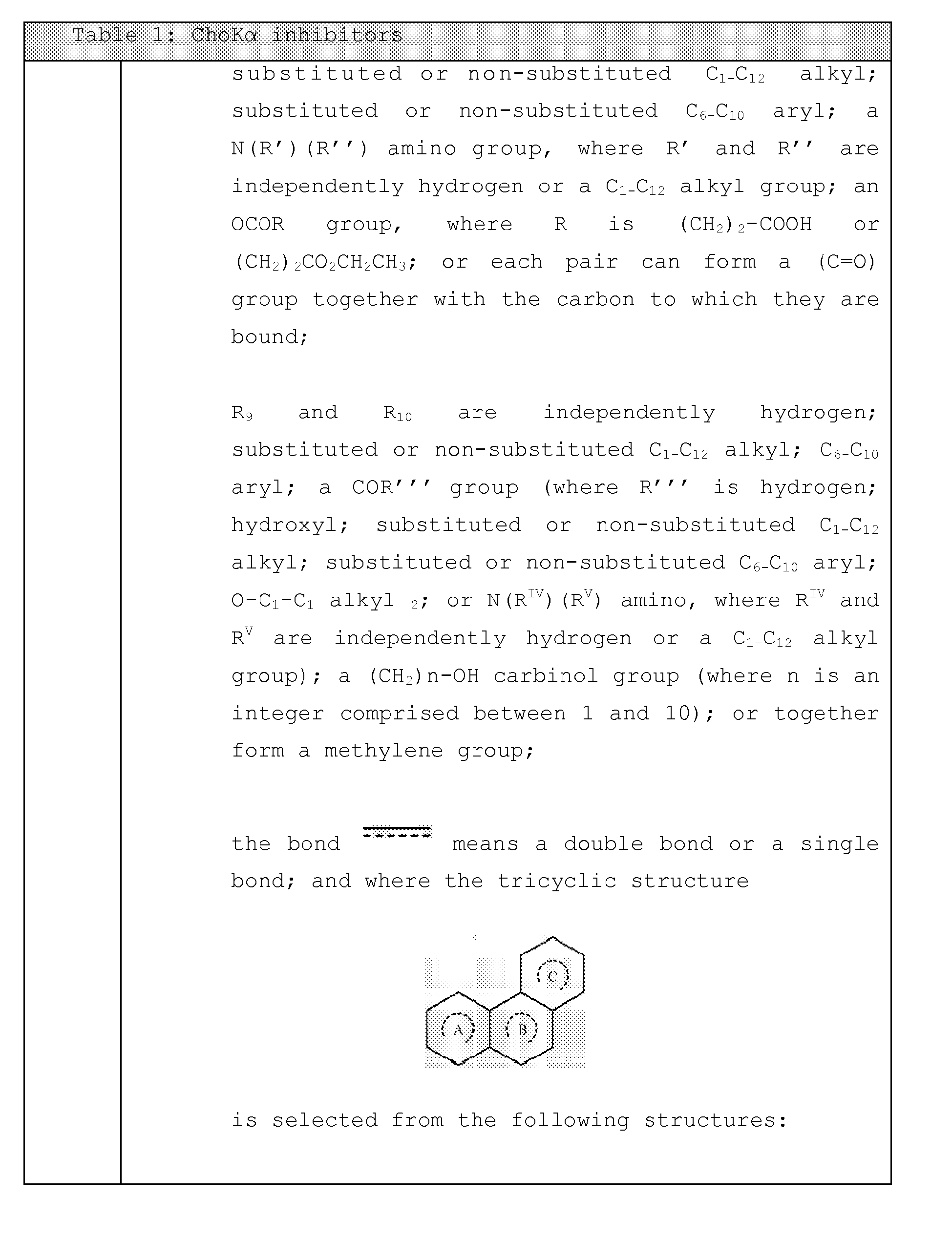

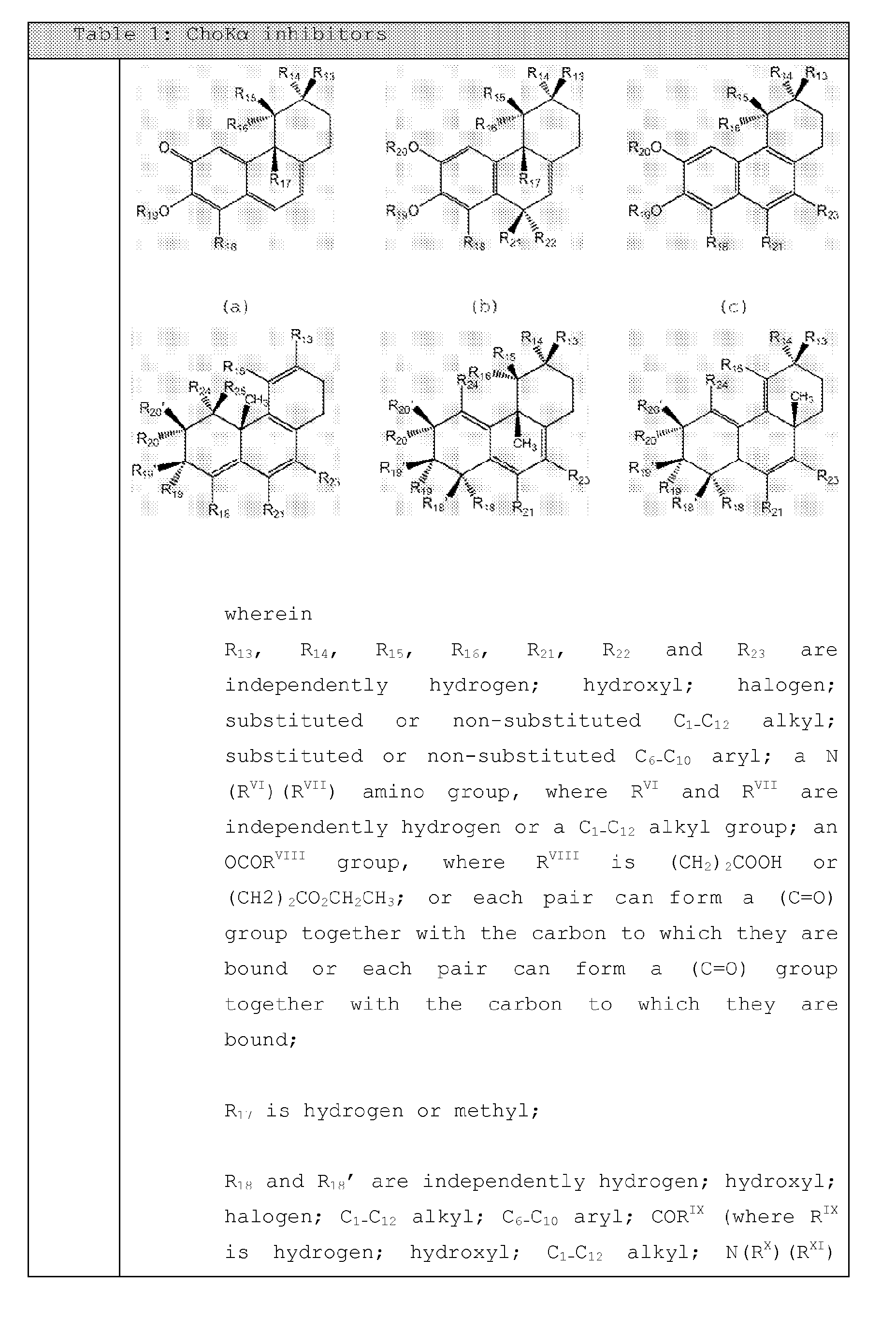

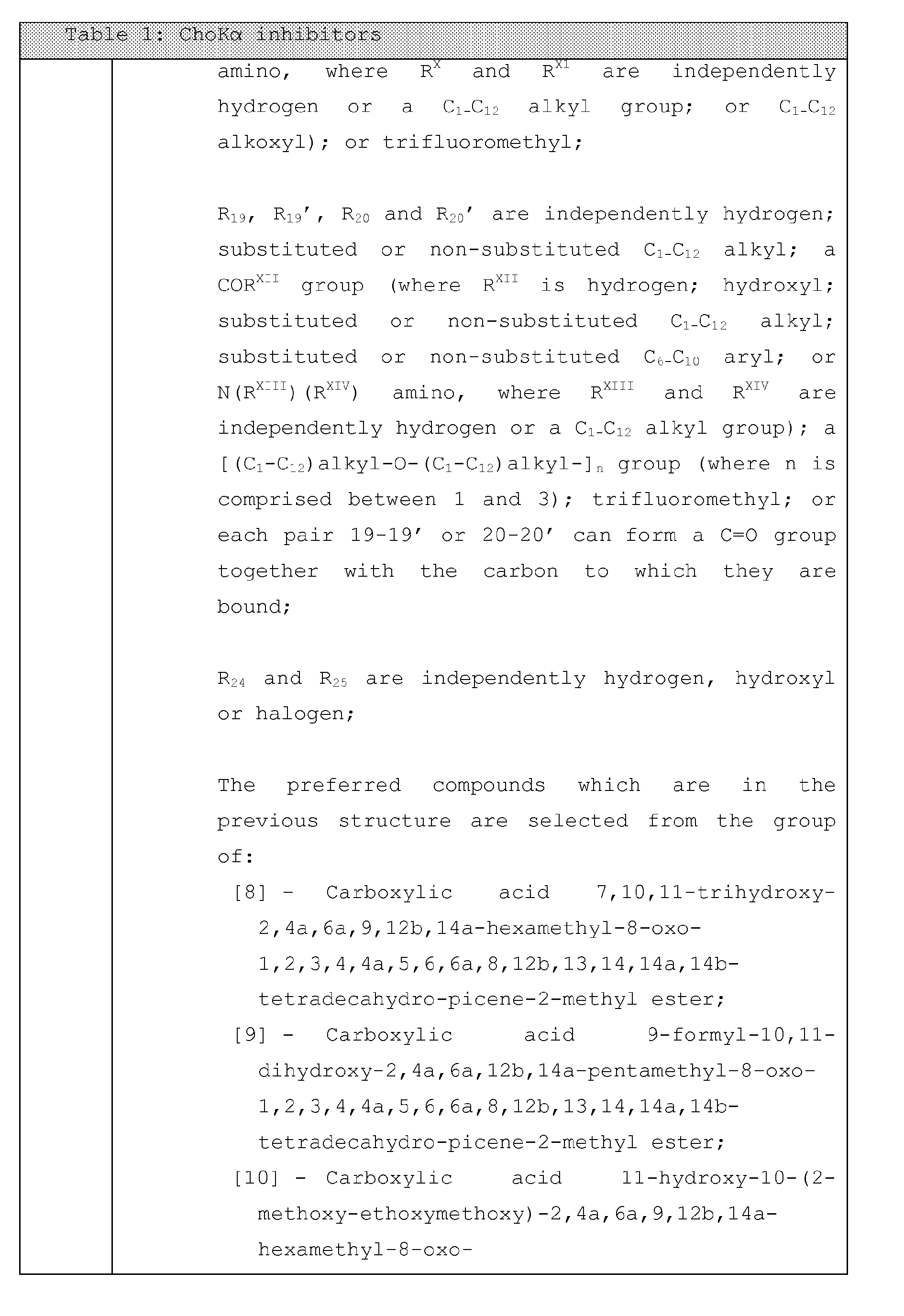

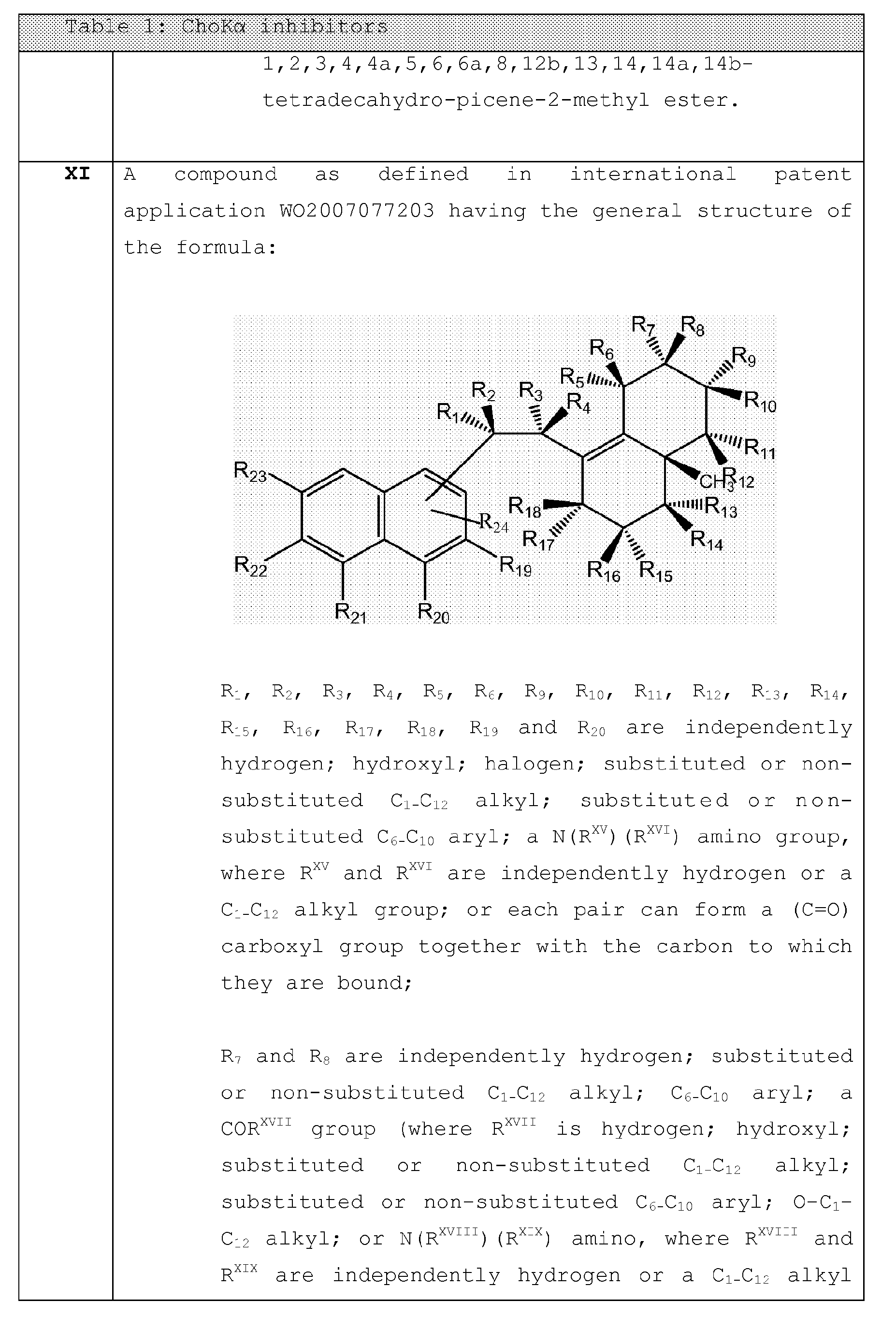

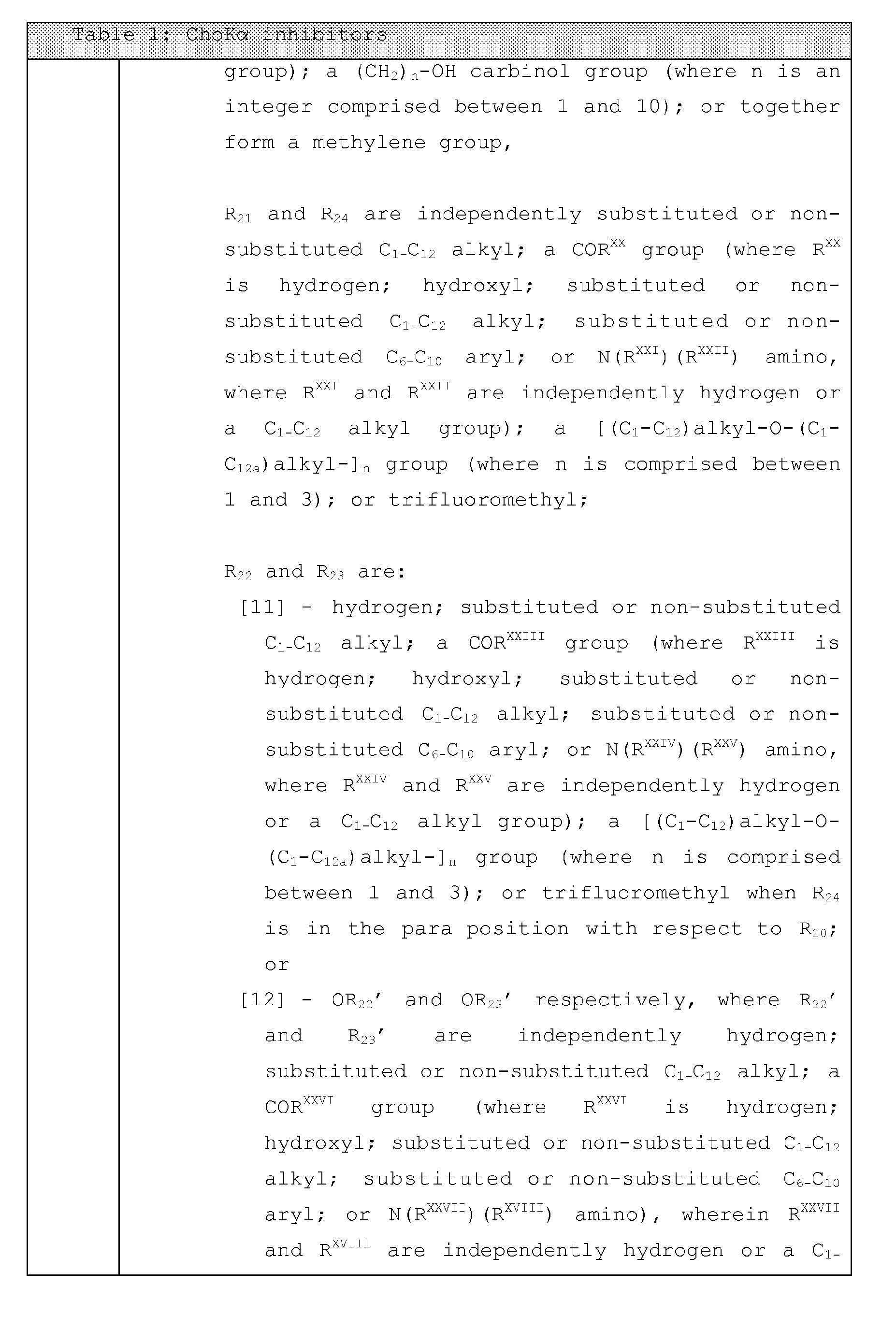

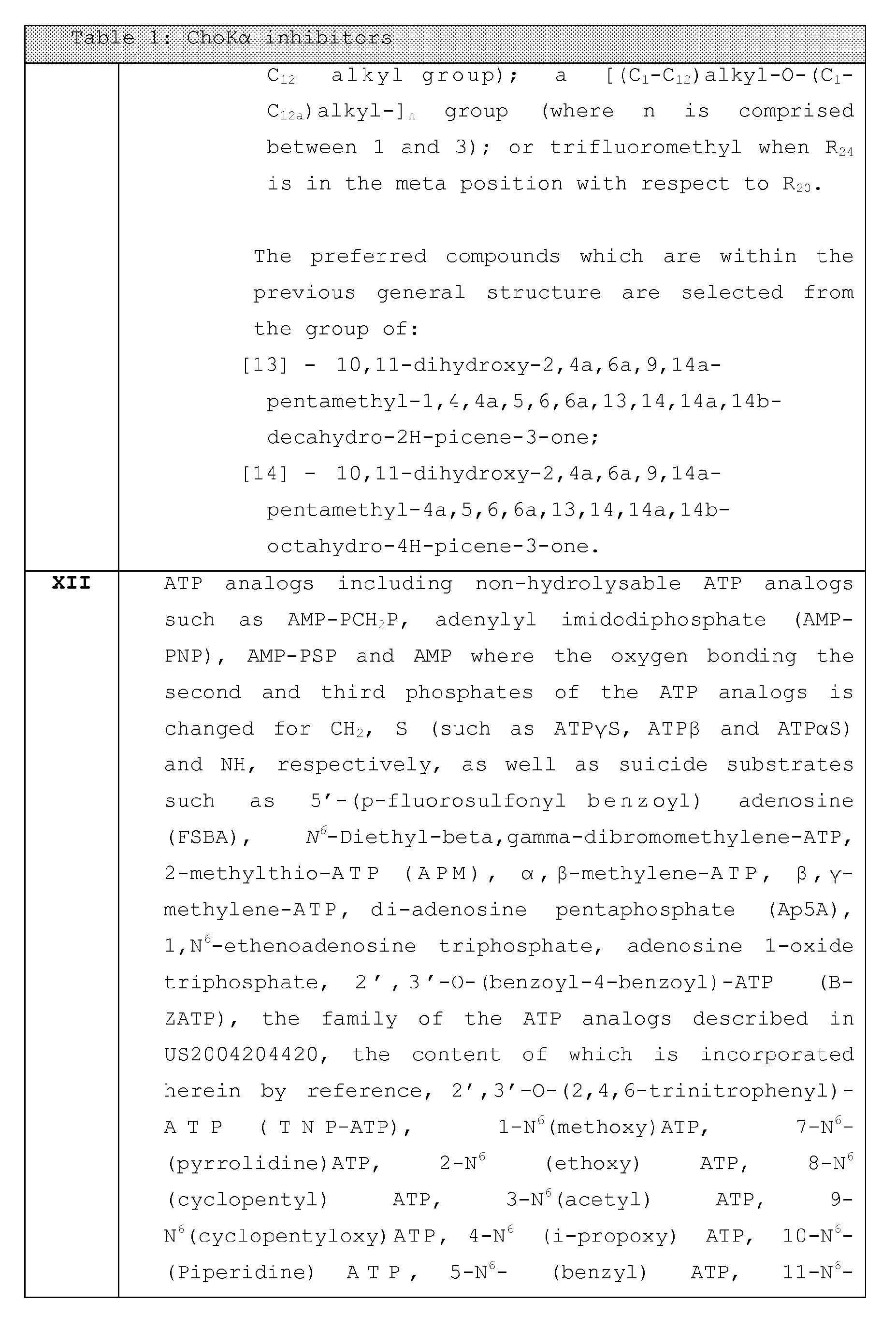

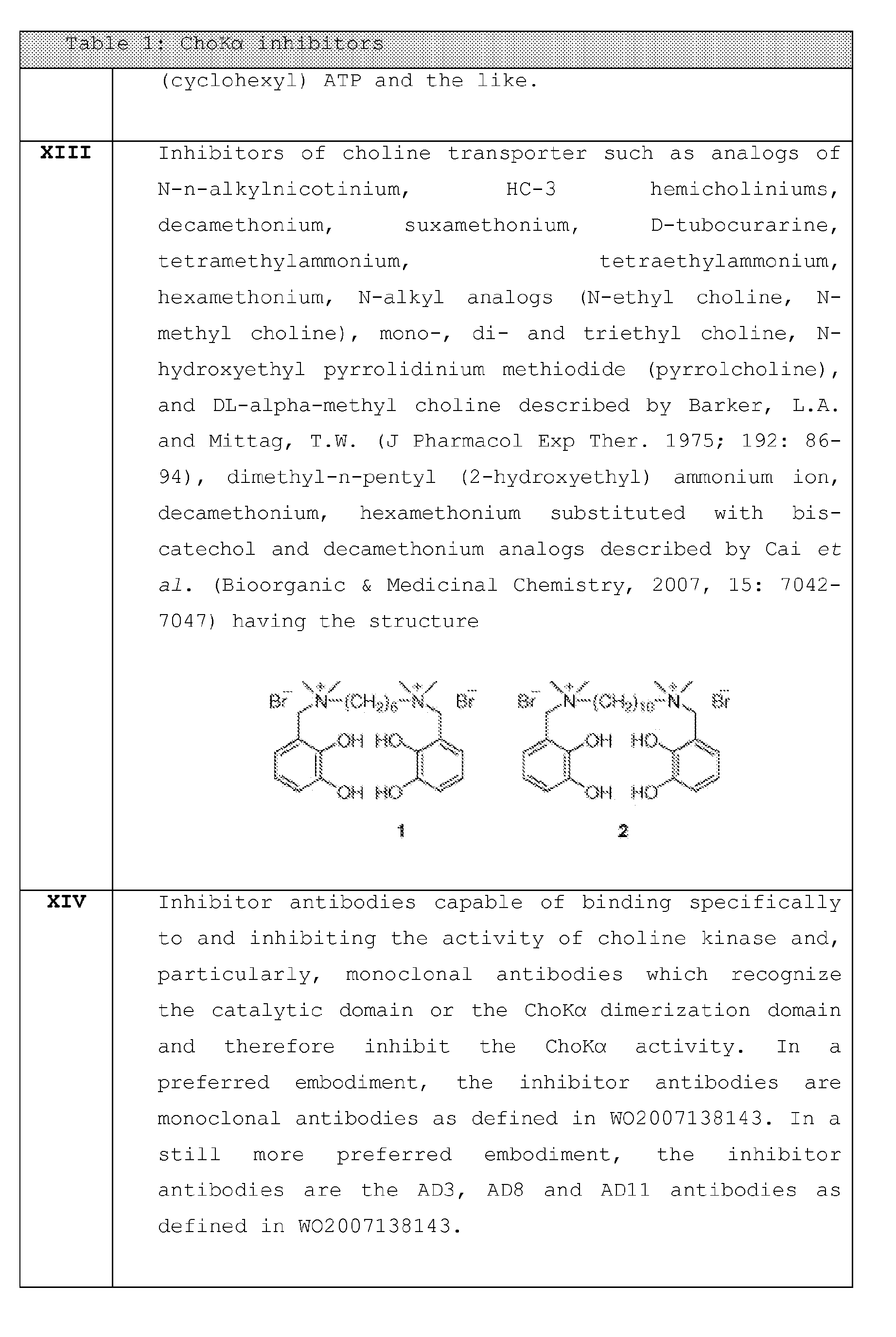

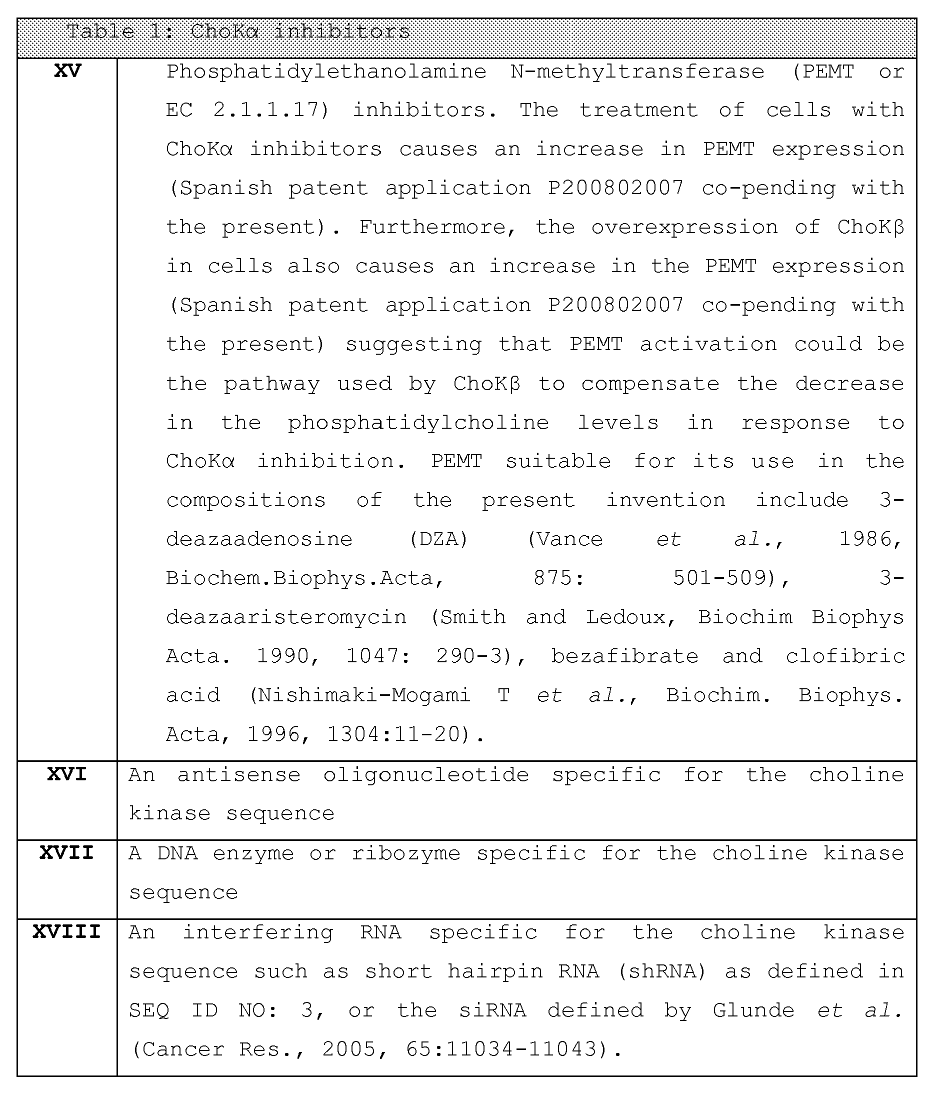

In a preferred embodiment, the method of personalized medicine of the invention is carried out in patients with cancer wherein the cancer is selected from the group of lung, breast, bladder or colorectal cancer.

### Methods of treatment of cancer based on the stimulation of ChoKβ activity

The authors of the present invention have surprisingly observed that ChoKβ expression in tumor cells results in a decrease in the proliferation rate of said cells. Thus, in Example 1.4 of the present invention it is demonstrated how ChoKβ and ChoKα overexpression in a cell results in the incidence of onset of tumors in comparison with the incidence of tumors resulting from ChoKα expression. Likewise, it has been observed that the implantation in athymic mice of tumor cells overexpressing ChoKβ and ChoKα gives rise to tumors having a volume which is 73% smaller than the tumors resulting from the implantation of cells expressing only ChoKα.

Thus, in a first aspect, the invention relates to a ChoKβ activity-inducing agent for its use in the treatment of cancer. Alternatively, the invention relates to the use of a ChoKβ activity-inducing agent for the preparation of a medicament for the treatment of cancer. Alternatively, the invention relates to a method of treatment of cancer in a subject comprising the administration to said individual of a ChoKβactivity-inducing agent.

In a preferred embodiment, the ChoKβ activity-inducing agent is selected from the group of:
(i) ChoKβ or a functionally equivalent variant of ChoKβ,
(ii) a polynucleotide encoding ChoKβ or a functionally equivalent variant thereof,
(iii)a vector comprising a polynucleotide according to (iii) and
(iv) a cell capable of secreting ChoKβ or a functionally equivalent variant thereof to the medium.

In the context of the present invention, ChoKβ is understood as a protein capable of phosphorylating choline into phosphorylcholine (PCho) and of phosphorylating the ethanolamine into phosphoethanolamine (PEtn) in the presence of magnesium (Mg2+), using adenosine 5'-triphosphate (ATP) as a phosphate group donor and which includes both the long variant of 395 aa (ChoKβ1) and the short variant of 127 aa (ChoKβ2) and which lacks choline/ethanolamine kinase domain, and which differs from the variant 1 in its C-terminal end resulting from an alternative splicing process.

ChoKβ polypeptides suitable for their use in the present invention include murine (accession number NCBI NP_031718 in the version of October 24, 2008), human (accession number NCBI NP_005189 in the version of June 28, 2009), rat (accession number NCBI NP_058873 in the version of October 24, 2008), zebrafish or *Danio rerio* (accession number NCBI NP_001093482 in the version of March 22, 2009) or *Xenopus laevis* (accession number NCBI NP_001011466 in the version of January 9, 2009) polypeptides.

In the context of the present invention, functionally equivalent variant of ChoKβ is understood as any molecule sharing with ChoKβ one or more of the functions described in the present invention associated to ChoKβ, both *in vitro* and *in vivo*, and having a minimum of identity in the amino acid sequence. Thus, ChoKβ variants suitable for their use in the present invention derive from the previously defined sequences by means of insertion, substitution or deletion of one or more amino acids and include natural alleles, variants resulting from alternative processing and naturally occurring secreted and truncated forms. Preferably, the ChoKβ variants preferably show an amino acid sequence identity with ChoKβ of at least 50%, at least 60%, at least 70%, at least 80%, at least 90%, at least 91%, at least 92%, at least 93%, at least 94%, at least 95%, at least 96%, at least 97%, at least 98% or at least 99%. The degree of identity is determined using methods well known for the persons skilled in the art. The identity between two amino acid sequences is preferably determined using the BLASTP algorithm [BLASTManual, Altschul, S., et al., NCBI NLM NIH Bethesda, Md. 20894, Altschul, S., et al., J. Mol. Biol. 215: 403-410 (1990)], preferably using the default parameters. In addition, the ChoKβ variants contemplated show at least some of the ChoKβ functions such as, without limitation:
- The capacity to inhibit the tumor proliferation of cells overexpressing ChoKα, for which the methods described in Example 1.4 of the present invention can be used,
- The capacity to promote an increase in the levels of phosphoethanolamine (PEtn) when it is expressed in a cell in the absence of ChoKα or to cause an increase in the levels of PEtn when it is expressed together with ChoKα greater than that observed when only ChoKα is expressed, for which the methods described in Example 1.4 of the present invention can be used.

As used in the present invention, the term "polynucleotide" relates to a nucleotide polymer form of any length and formed by ribonucleotides and/or deoxyribonucleotides. The term includes both single-stranded and double-stranded polynucleotides, as well as modified polynucleotides (methylated, protected polynucleotides and the like).

Polynucleotides suitable for their use as agents capable of inducing ChoKα activity include, without limitation, the polynucleotides the sequences of which correspond to human ChoKβ mRNA (accession number NM_005198 in NCBI in the version of June 28, 2009), mouse ChoKβ mRNA (accession number NM_007692 in NCBI in the version of October 24, 2008), rat ChoKβ mRNA (accession number NM_017177 in NCBI in the version of October 24, 2008), zebrafish ChoKβ mRNA (accession number NM_001100012 in NCBI in the version of March 22, 2009).

Alternatively, the agents capable of inducing ChoKβ activity include functionally equivalent variants of the polynucleotides previously defined by means of their specific sequences. In the context of the present invention, "functionally equivalent polynucleotide" is understood as all those polynucleotides capable of encoding a polypeptide with ChoKβ activity, as has been previously defined, and which result from the previously defined polynucleotides by means of insertion, deletion or substitution of one or several nucleotides with respect to the previously defined sequences. The variant polynucleotides of the present invention are preferably polynucleotides the sequence of which allows them to hybridize in highly stringent conditions with the previously defined polynucleotides. Typical highly stringent hybridizing conditions include the incubation in 6 X SSC (1 X SSC: 0.15 M NaCl, 0.015 M sodium citrate) and 40% formamide at 42°C for 14 hours, followed by one or several washing cycles using 0.5 X SSC, 0.1% SDS at 60°C. Alternatively, highly stringent conditions include those comprising a hybridization at a temperature of approximately 50°-55°C in 6 X SSC and a final washing at a temperature of 68°C in 1-3 X SSC. Moderate stringent conditions comprise the hybridization at a temperature of approximately 50°C to about 65°C in 0.2 or 0.3 M NaCl, followed by washing at approximately 50°C to about 55°C in 0.2 X SSC, 0.1% SDS (sodium dodecyl sulfate).

Preferably, when the agent which is capable of inducing ChoKβ activity is a polynucleotide, the latter is operatively bound to an expression regulatory region. The regulatory sequences useful for the present invention can be nuclear promoter sequences or, alternatively, enhancer sequences and/or other regulatory sequences increasing the heterologous nucleic acid sequence expression. The promoter can be constitutive or inducible. If constant heterologous nucleic acid sequence expression is desired, then a constitutive promoter is used. Examples of well known constitutive promoters include the cytomegalovirus (CMV) immediate-early promoter, Rous sarcoma virus promoter and the like. A number of other examples of constitutive promoters are well known in the art and can be used in the practice of the invention. If controlled heterologous nucleic acid sequence expression is desired, then an inducible promoter must be used. In a non-induced state, the inducible promoter is "silent". By "silent" it is meant that in the absence of an inducer little or no heterologous nucleic acid sequence expression is detected; in the presence of an inducer, however, heterologous nucleic acid sequence expression occurs. Often, the expression level can be controlled varying the concentration of the inducer. Controlling the expression, for example varying the concentration of the inducer such that an inducible promoter is more strongly or more weakly stimulated, the concentration of the transcript product of the heterologous nucleic acid sequence can be affected. In the event that the heterologous nucleic acid sequence encodes a gene, the amount of protein which is synthesized can be controlled. It is thus possible to vary the concentration of the therapeutic product. Examples of well known inducible promoters are: an estrogen or androgen promoter, a metallothionein promoter, or a promoter which responds to ecdysone. A number of other examples are well known in the art and can be used in the practice of the invention. In addition to the constitutive and inducible promoters (which usually work in a large variety of types of cells or tissues), tissue-specific promoters can be used to achieve specific heterologous nucleic acid sequence expression in cells or tissues. Well known examples of tissue-specific promoters include several muscle-specific promoters including: the skeletal α-actin promoter, the cardiac actin promoter, skeletal troponin C promoter, cardiac/slow-twitch troponin C promoter and the creatine kinase promoter/enhancer. There are a number of muscle-specific promoters which are well known in the art and which can be used in the practice of the invention (for a review on muscle-specific promoters, see Miller et al., (1993) Bioessays 15: 191-196).

In another embodiment, the ChoKβ activity-inducing agent is a vector comprising a polynucleotide as has been previously described, i.e., encoding ChoKβ or a functionally equivalent variant thereof. Vectors suitable for the insertion of said polynucleotides are vectors derived from expression vectors in prokaryotes such as pUC18, pUC19, pBluescript and derivatives thereof, mp18, mp19, pBR322, pMB9, ColE1, pCRl, RP4, phages and shuttle vectors such as pSA3 and pAT28, expression vectors in yeasts such as vectors of the type of 2 micron plasmids, integrative plasmids, YEP vectors, centromere plasmids and the like, expression vectors in cells of insects such as the vectors of the pAC series and of the pVL series, expression vectors in plants such as vectors of the pIBI, pEarleyGate, pAVA, pCAMBIA, pGSA, pGWB, pMDC, pMY, pORE series and the like and expression vectors in higher eukaryotic cells based on viral vectors (adenoviruses, viruses associated to adenoviruses as well as retroviruses and, particularly, lentiviruses) as well as non-viral vectors such as pSilencer 4.1-CMV (Ambion), pcDNA3, pcDNA3.1/hyg pHCMV/Zeo, pCR3.1, pEFl/His, pIND/GS, pRc/HCMV2, pSV40/Zeo2, pTRACER-HCMV, pUB6/V5-His, pVAX1, pZeoSV2, pCI, pSVL and pKSV-10, pBPV-1, pML2d and pTDT1.

In another embodiment, the ChoKβ activity-inducing agent is a cell capable of secreting ChoKβ or a functionally equivalent variant thereof to the medium. Cells suitable for the expression of ChoKβ or of the functionally equivalent variant thereof include, without limitation, cardiomyocytes, adipocytes, endothelial cells, epithelial cells, lymphocytes (B and T cells), mastocytes, eosinophils, vascular intima cells, primary cultures of cells isolated from different organs, preferably from cells isolated from islets of Langerhans, hepatocytes, leukocytes, including mononuclear, mesenchymal, umbilical cord or adult (skin, lung, kidney and liver) leukocytes, osteoclasts, chondrocytes and other cells of the connective tissue. Established cell lines such as Jurkat T cells, NIH-3T3 cells, CHO, Cos, VERO, BHK, HeLa, COS, MDCK, 293, 3T3, C2C12 myoblasts and W138 cells are also suitable.

The person skilled in the art will understand that the cells capable of secreting ChoKβ or a functionally equivalent variant thereof to the medium can be found forming microparticles or microcapsules such that the cells have a longer useful life before being used in patients. Materials suitable for the formation of the microparticles object of the invention include any biocompatible polymer material allowing the continuous secretion of the therapeutic products and acting as a cell support. Thus, said biocompatible polymer material can be, for example, thermoplastic polymers or hydrogel polymers. Thermoplastic polymers include acrylic acid, acrylamide, 2-aminoethyl methacrylate, poly(tetrafluoroethylene-cohexafluoropropylene), methacrylic acid-(7-coumaroxy) ethyl ester, N-isopropyl acrylamide, polyacrylic acid, polyacrylamide, polyamidoamine, poly(amino)-p-xylylene, poly(chloroethyl vinyl ether), polycaprolactone, poly(caprolactone-co-trimethylene carbonate), poly(carbonate-urea) urethane, poly(carbonate) urethane, polyethylene, archylamide and polyethylene copolymers, polyethylene glycol, polyethylene glycol methacrylate, poly(ethylene terephthalate), poly(4-hydroxybutyl acrylate), poly(hydroxyethyl methacrylate), poly(N-2-hydroxypropyl methacrylate), poly(lactic acid-glycolic acid), poly(L lactic acid), poly(gamma-methyl, L-glutamate), poly(methylmethacrylate), poly(propylene fumarate), poly(propylene oxide), polypyrrole, polystyrene, poly(tetrafluoroethylene), polyurethane, polyvinyl alcohol, ultra high molecular weight polyethylene, 6-(p-vinylbenzamido)-hexanoic acid and N-p-vinylbenzyl-D-maltonamide and copolymers containing more than one of said polymers. Polymers of the hydrogel type include natural materials of the type of alginate, agarose, collagen, starch, hyaluronic acid, bovine serum albumin, cellulose and derivatives thereof, pectin, chondroitin sulfate, fibrin and fibroin, as well as synthetic hydrogels such as sepharose and sephadex.

It is known from the state of the art that some of the previously mentioned polymers are not very stable and tend to lose their gel character, in addition to being relatively porous, which results in the antibodies being able to enter inside them and damage the cells. For these reasons, the microparticle of the invention can optionally be surrounded by a semipermeable membrane conferring stability to the particles and forming a barrier impermeable to the antibodies. Semipermeable membrane is understood as a membrane which allows the entrance of all those solutes necessary for cell viability and which allow the exit of the therapeutic proteins produced by the cells contained inside the microparticle, but which is substantially impermeable to the antibodies, such that the cells are protected from the immune response caused by the organism housing the microparticle. Materials suitable for forming the semipermeable membrane are materials insoluble in biological fluids, preferably polyamino acids, such as for example poly-L-lysine, poly-L-ornithine, poly-L-arginine, poly-L-asparagine, poly-L-aspartic, poly benzyl-L-aspartate, poly-S-benzyl-L-cysteine, poly-gamma-benzyl-L-glutamate, poly-S-CBZ-L-cysteine, poly-s-CBZ-D-lysine, poly-δ-CBZ-DL-ornithine, poly-O-CBZ-L-serine, poly-O-CBZ-D-tyrosine, poly(γ-ethyl-L-glutamate), poly-D-glutamic, polyglycine, poly-γ-N-hexyl L-glutamate, poly-L-histidine, poly(α,β-[N-(2-hydroxyethyl)-DL-aspartamide]), poly-L-hydroxyproline, poly (α,β-[N-(3-hydroxypropyl)-DL-aspartamide]), poly-L-isoleucine, poly-L-leucine, poly-D-lysine, poly-L-phenylalanine, poly-L-proline, poly-L-serine, poly-L-threonine, poly-DL-tryptophan, poly-D-tyrosine or a combination thereof.

In the context of the invention, "treatment of cancer" means the combined administration of a composition according to the invention to prevent or delay the onset of symptoms, complications or biochemical indications of cancer or tumor, to alleviate its symptoms or to stop or inhibit its development and progression such as, for example, the onset of metastasis. The treatment can be a prophylactic treatment to delay the onset of the disease or to prevent the manifestation of its clinical or subclinical symptoms or a therapeutic treatment to eliminate or alleviate the symptoms after the manifestation of the disease or in relation to its surgical or radiotherapy treatment.

The cancer that will be treated in the context of the present invention can be any type of cancer or tumor. These tumors or cancer include, and are not limited to, hematological cancers (for example leukemias or lymphomas), neurological tumors (for example astrocytomas or glioblastomas), melanoma, breast cancer, lung cancer, head and neck cancer, gastrointestinal tumors (for example stomach, pancreatic or colorectal cancer), liver cancer (for example hepatocellular carcinoma), renal cell cancer, genitourinary tumors (for example ovarian cancer, vaginal cancer, cervical cancer, bladder cancer, testicular cancer, prostate cancer), bone tumors and vascular tumors. Therefore, in a particular embodiment, the cancer disease that will be treated or prevented is a lung, breast, bladder or colorectal cancer.

In the context of the present invention, "lung cancer" is understood as any type of tumor damage of the lung tissue, including non-small cell cancer or NSCLC. In an even more particular embodiment, the NSCLC is selected from squamous cell lung carcinoma, large cell lung carcinoma and lung adenocarcinoma. Furthermore, the present method is also applicable to a subject who suffers from any NSCLC stage (stages 0, IA, IB, IIA, IIB, IIIA, IIIB or IV).

In the context of the present invention, the term "breast cancer" is understood as any type of tumor damage of the breast and includes, without limitation, ductal carcinoma *in situ* (DCIS), infiltrating or invasive ductal carcinoma, lobular carcinoma *in situ* (LCIS), infiltrating or invasive lobular carcinoma and inflammatory carcinoma and includes tumors in stages 0, I, II, IIIA, IIIB, IIIC and IV.

The term "bladder cancer" relates to a tumor of the bladder and includes any subtype with a histology which typically occurs in bladder cancer such as transitional cell carcinoma, squamous cell carcinoma and adenocarcinoma, any clinical subtype such as superficial muscle-invasive cancer or metastatic disease and any TNM stage including tumors T0-T4, N0-N4 and M0-M4.

As used herein, the term "colorectal cancer" includes any type of neoplasia of the colon, rectum and appendix and refers to both early and late adenomas and carcinomas as well as to hereditary, familial or sporadic cancer. Hereditary CRC includes those syndromes which include the presence of polyps, such as hamartomatous polyposis syndromes and the most known, familial adenomatous polyposis (FAP) as well as non-polyposis syndromes such as hereditary nonpolyposis colorectal cancer (HNPCC) or Lynch syndrome 1. Likewise, the invention contemplates the treatment of colorectal cancer in its different stages such as stages A, B, C1, C2 and D according to the Dukes classification, stages A, B1, B2, B3, C1, C2, C3 and D according to the Astler-Coller classification, stages TX, T0, Tis, TI, T2, T3, NX, NO, NI, N2, MX, MO and MI according to the TNM system as well as stages 0, I, II, III and IV according to the AJCC (American Joint Committee on Cancer) classification.

The compositions of the invention have demonstrated to be particularly efficient for the treatment of tumors in which there are high ChoKa expression levels. As used herein, the expression "high ChoKα expression levels" relates to levels of ChoKα greater than those observed occurring in a reference sample. In particular, it can be considered that a sample has high ChoKα expression levels when the expression levels are at least 1.1 times, 1.5 times, 5 times, 10 times, 20 times, 30 times, 40 times, 50 times, 60 times, 70 times, 80 times, 90 times, 100 times or even more with respect to said reference sample.

Said reference sample is typically obtained combining equal amounts of samples from a population of subjects. The typical reference samples will generally be obtained from subjects who are clinically well documented and who are disease-free. In such samples, the normal (reference) concentrations of the biomarker can be determined, for example providing the mean concentration over the reference population. When the reference concentration of the marker is determined, several considerations are taken into account. Such considerations include the type of sample involved (for example tissue or CSF), age, weight, sex, general physical condition of the patient and the like. For example, equal amounts of a group of at least 2, at least 10, at least 100 to preferably more than 1000 subjects, preferably classified according to the previous considerations, for example of several categories of age are taken as a reference group.

The determination of the ChoKα expression levels both in the sample of the tumor to be treated and in the reference sample can be carried out determining the levels of mRNA encoded by ChoKα using conventional techniques such as RT-PCR, RNA protection analysis, Northern procedure, *in situ* hybridization, microarray technology and the like or determining the levels of the ChoKα protein, using to that end conventional techniques of the type of immunoblot or Western blot, ELISA (adsorption enzyme immunoanalysis), RIA (radioimmunoassay), competitive EIA (competitive enzyme immunoassay), DAS-ELISA (double antibody sandwich ELISA), immunocytochemical and immunohistochemical techniques, techniques based on the use of biochip or protein microarrays which include specific antibodies or assays based on colloidal precipitation in formats such as reagent strips.

The compounds of the invention can be administered both in acute form and in chronic form. As used in the present invention, the expression "chronic administration" relates to a method of administration in which the compound is administered to the patient continuously during extended time periods for the purpose of maintaining the therapeutic effect during said period. Chronic administration form includes the administration of multiple doses of the compound daily, twice a day, three times a day or with a lower frequency. The chronic administration can be carried out by means of several intravenous injections administered periodically throughout a single day. Alternatively, the chronic administration involves the administration in bolus form or by means of continuous transfusion which can be carried out daily, every two days, every 3 to 15 days, every 10 days or more. Typically, the chronic administration is maintained for at least 72 hours, at least 96 hours, at least 120 hours, at least 144 hours, at least 1 week, at least 2 weeks, at least 3 weeks, at least 4 weeks, at least 5 weeks, at least 6 weeks, at least 7 weeks, at least 8 weeks, at least 9 weeks, at least 10 weeks, at least 11 weeks, at least 12 weeks, at least 4 months, at least 5 months, at least 6 months, at least 9 months, at least a year, at least 2 years or more.

As used in the present invention, the expression "acute administration" relates to a method of administration in which the patient is exposed to a single dose of the compound or to several doses but during a reduced time period such as for example, 1, 2, 4, 6, 8, 12 or 24 hours or 2, 3, or 4 days.

The person skilled in the art will understand that the therapeutically effective amount, and/or formulation of the active compound will be carried out depending on the type of administration. As used herein, "therapeutically effective amount" means the amount of compound which allows completely or partially eliminating the tumor growth.

In the event that a chronic administration of the compound of the invention is desired, it can be administered in a sustained release composition such as that described in documents US5672659, US5595760, US5821221, US5916883 and WO9938536. In contrast, if acute administration is desired, a treatment with an immediate release form will be preferred. Regardless of the type of administration, the dosage amount and the interval can be adjusted individually to provide plasma levels of the compounds which are sufficient to maintain the therapeutic effect. A person having ordinary skill in the art will be capable of optimizing the therapeutically effective local doses without too much experimentation.

The administration of the compounds of the invention requires their formulation in pharmaceutical compositions, which constitute another aspect of the invention. The pharmaceutical compositions useful in the practice of the method of the invention include a therapeutically effective amount of an active agent, and a pharmaceutically acceptable carrier. The term "pharmaceutically acceptable" means approved by a regulatory agency of a state or federal government or included in the United States Pharmacopoeia or other generally recognized pharmacopoeia, for use in animals, and more particularly in humans. The term "carrier" relates to a diluent, coadjuvant, excipient, or vehicle with which the therapeutic compound is administered. Such pharmaceutical carriers can be sterile liquids, such as water and oils, including petroleum, animal, plant or synthetic oils, such as peanut oil, soybean oil, mineral oil, sesame oil and the like. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain smaller amounts of wetting agents or emulsifiers, or pH buffering agents. These compositions can take the form of solutions, suspensions, emulsions, tablets, pills, capsules, powders, prolonged release formulations and the like. The composition can be formulated as a suppository, with traditional binders and carriers such as triglycerides. The oral formulation can include standard carriers such as pharmaceutical types of mannitol, lactose, starch, magnesium stearate, sodium saccharin, cellulose, magnesium carbonate, etc. Examples of suitable pharmaceutical carriers are described in "Remington's Pharmaceutical Sciences" by E.W. Martin.

In the event that nucleic acids are administered (the polynucleotides of the invention, the vectors or the gene constructs), the invention contemplates pharmaceutical compositions especially prepared for the administration of said nucleic acids. The pharmaceutical compositions can comprise said nucleic acids in naked form, i.e., in the absence of compounds protecting the nucleic acids from their degradation by the nucleases of the organism, which involves the advantage of eliminating the toxicity associated with the reagents used for the transfection. Suitable routes of administration for the naked compounds include intravascular, intratumoral, intracranial, intraperitoneal, intrasplenic, intramuscular, subretinal, subcutaneous, mucosal, topical and oral route (Templeton, 2002, DNA Cell Biol., 21:857-867). Alternatively, the nucleic acids can be administered forming part of liposomes, conjugated to cholesterol or conjugated to compounds capable of promoting the translocation through cell membranes such as the Tat peptide derived from the HIV-1 TAT protein, the third helix of the homeodomain of the Antennapedia protein of *D. melanogaster,* the VP22 protein of the herpes simplex virus, arginine oligomers and peptides such as those described in WO0706909 (Lindgren, A. et al., 2000, Trends Pharmacol. Sci, 21:99-103, Schwarze, S.R. et al., 2000, Trends Pharmacol. Sci., 21:45-48, Lundberg, M et al., 2003, Mol. Therapy 8:143-150 and Snyder, E.L. and Dowdy, S.F., 2004, Pharm. Res. 21:389-393). Alternatively, the polynucleotide can be administered forming part of a plasmid vector or of a viral vector, preferably vectors based on adenoviruses, on adeno-associated viruses or on retroviruses, such as viruses based on the murine leukemia virus (MLV) or on lentiviruses (HIV, FIV, EIAV).

The composition can be formulated according to routine procedures as a pharmaceutical composition adapted for intravenous, subcutaneous or intramuscular administration to human beings. When necessary, the composition can also include a solubilizing agent and a local anesthetic such as lidocaine to alleviate the pain in the injection site. When the composition is to be administered by infiltration, it can be dispensed with an infiltration flask containing water or saline solution of pharmaceutical quality. When the composition is administered by injection, an ampoule of water for injection or sterile saline solution can be provided, therefore the ingredients can be mixed before the administration.

The amount of the ChoKβ activity-inducing compound which will be effective in the treatment of cancer can be determined by clinical standard techniques based on the present description. Furthermore, *in vitro* assays can be optionally used to aid in identifying the optimum dosage intervals. The precise dose to be used in the formulation will also depend on the route of administration, and the severity of the disease, and it must be decided according to the judgment of the doctor and the circumstances of each subject. However, suitable dose intervals for intravenous administration are generally approximately 50-5000 micrograms of active compound per kilogram of body weight. The suitable dosage intervals for intranasal administration are generally approximately 0.01 pg/kg of body weight to 1 mg/kg of body weight. The effective dose can be extrapolated from dose response curves derived from *in vitro* or animal assay model systems.

For the systemic administration, a therapeutically effective dose can be initially estimated from *in vitro* assays. For example, a dose can be formulated in animal models to achieve a circulating concentration interval including the IC50 which has been determined in cell culture. Such information can be used to determine the useful dose in humans more precisely. The initial doses can also be estimated from *in vivo* data, e.g., animal models, using techniques which are well known in the art. A person having ordinary skill in the art may easily optimize the administration to humans based on the data in animals.

### Other aspects of the invention

In additional aspects, the invention relates to:
[1] - A Chokβ enzyme expression vector to be used in a gene therapy method inhibiting the Chokα enzyme intended for the treatment of cancer.
[2] - A Chokβ enzyme expression vector to be used in a gene therapy method inhibiting the Chokα enzyme intended for the treatment of lung, breast, bladder or colorectal cancer.
[3] - An expression vector according to [1] or [2], characterized by being a virus.
[4] - Use of a Chokβ enzyme expression vector as an antioncogenic Chokα enzyme-inhibiting agent.
[5] - Use of a Chokβ enzyme expression vector for preparing gene therapy compositions inhibiting the Chokα enzyme expression intended for the treatment of cancer.
[6] - Use according to [5], characterized in that the cancer is lung, breast, bladder or colorectal cancer.
[7] - Use according to [4] to [6], characterized in that the vector is a virus.
[8] - A gene therapy composition characterized by comprising a Chokβ enzyme expression vector capable of inhibiting the Chokα enzyme.
[9] - A cell transfected by a Chokβ enzyme expression vector characterized by having a reduced Chokα expression level and/or by its incapacity to grow or proliferate in a pathological manner.

The invention is illustrated below by means of the following examples which must be considered as merely illustrative and in no case as limiting the scope of the invention.

### EXAMPLES

### EXAMPLE 1

### USE OF CHOKβ AS A TUMOR SUPPRESSOR

### 1.1. ChoKα and ChoKβ gene expression profile in human cell lines

For the purpose of determining if there are differences in the alteration of the expression of the different ChoK isoforms in cancer, the endogenous mRNA expression of ChoKα and ChoKβ in a panel of cell lines derived from human breast, bladder, colorectal and lung tumors was verified in the present invention by means of quantitative PCR. Each tumor type was compared in turn with its corresponding non-transformed primary parent lines as a control.

The levels of ChoKα and ChoKβ mRNA of various human small cell and non-small cell lung cancer tumor lines were compared with the primary lung line (BEC). The results shown in Figure 1A indicate that there is only an overexpression pattern of the ChoKα messenger in all the tumor lines compared with the senescent primary line. Moreover, in the case of the ChoKβ isoform an opposite pattern is observed, i.e. a silencing of the expression of this protein in the tumor lines with respect to the primary line. Similar results were obtained in the human bladder tumor lines compared with the UROTsa non-transformed line (Figure 1B), in which the levels of ChoKα messenger are overexpressed whereas those of ChoKβ remain silenced in the tumor lines.

Like in the previously described cases, in the tumor lines derived from human breast cancer increased levels of the ChoKα isoform were found against a senescent primary epithelial line (HMEC), whereas no significant difference was found in the ChoKβ expression pattern (Figure 1C).

These results demonstrate that several tumor lines have as a common characteristic the increase of ChoKα gene expression whereas ChoKβ is not affected, suggesting that the high levels of the ChoKα isoform are relevant for the tumor process, this not being the case for ChoKβ the levels of which are even reduced in some of the cases.

### 1.2. ChoKα and ChoKβ gene expression profile in samples of patients

In the same way as carried out for the case of human tumor lines, the expression levels of the α and β isoforms of ChoK were studied in a series of 33 samples of patients diagnosed with lung cancer. To that end, the levels of ChoKα and ChoKβ were determined by means of quantitative PCR, comparing them with commercial normal human lung tissue RNA as a reference.

The results of the quantitative PCR reproduce the data obtained previously for the cell lines, an increase of more than two times of the ChoKα expression levels in the tumor samples with respect to the normal tissue being obtained in 39.4% (Figure 2A). For the case of the ChoKβ isoform (Figure 2B), a reduction of more than two times of the expression levels was obtained in 66.7% of the tumor samples compared with the normal tissue, similarly to the results found in the cell lines.

These data indicate that the α and β isoforms of ChoK have an opposite behavior in cell transformation conditions, suggesting a differential role for both proteins in the carcinogenic process.

### 1.3. Induction of the ChoKβ expression in response to MN58b

In the present invention, the sensitivity of ChoKβ to the chemical inhibitor MN58b was furthermore estimated, concluding that the ChoKα isoform is markedly more sensitive to the antiproliferative effect of MN58b than the ChoKβ isoform. As a result, in conditions in which the treatment with this drug is inducing cell death, only the ChoKα isoform is affected. For the purpose of verifying that a compensation effect of the function of ChoKα by ChoKβ is not occurring, the transcriptional response of ChoKβ to the pharmacological inhibition of ChoKα with MN58b was studied. To carry out this study, a panel of human tumor cells of different origins having an efficient *in vitro* response to the antiproliferative effect of the treatment with MN58b, including Hek293T, Jurkat, H1299 and SW780, was chosen. The cells were treated with 20 µM MN58b (concentration at which ChoKα is inhibited but ChoKβ is not significantly affected) for 24 and 48 hours, and the effect of the drug was verified by means of immunodetection of PARP proteolysis or Caspase 3 degradation as indicators of cell death (Figure 3). In addition, the human ChoKβ levels were also determined by means of quantitative PCR. As shown in Figure 4, in all the cases there is an increase of the levels of ChoKβ in response to MN58b, although the time of maximum induction is different for each cell line.

These results suggest that the regulation of both ChoK isoforms is related, ChoKβ being transcriptionally induced in response to the pharmacological inhibition of ChoKα.

### 1.4. Regulatory role of ChoKβ in the transformation mediated by ChoKα

ChoKα overexpression but not ChoKβ overexpression induces transformation in human Hek293T cells. In addition, various cell lines derived from human tumors and samples of patients with lung cancer have high levels of ChoKα mRNA and reduced levels of ChoKβ mRNA with respect to their corresponding normal controls. This suggests a different but linked behavior of both isoforms in the cell transformation process.

To study the possible combined regulation of the ChoK isoforms, the intracellular levels of PCho and PEtn generated upon overexpressing both isoforms together were analyzed. To that end, Hek293T cells were transfected with the empty pCDNA3b vector as a control, and the expression vectors encoding ChoKα, ChoKβ, or both together, and labeled *in vitro* at equilibrium with ¹⁴C-choline or ¹⁴C-ethanolamine. The results shown in Figure 5 confirm that while ChoKα is capable of promoting high levels of PCho and of PEtn, ChoKβ preferably participates in the induction of levels of PEtn. In the case of the joint overexpression of both isoforms, there is an increase of the intracellular levels of PEtn above those obtained with each of the two isoforms separately. However, the levels of PCho are reduced with respect to those obtained with ChoKα overexpression, although they still remain greater than the control. In accordance with these results, with regard to the dimerization properties of the different ChoK isoforms forming α/α, β/β homodimers or α/β heterodimers, it has been previously demonstrated that different degrees of ChoK activity are generated, the most active dimers being those formed by α/α molecules, and the least active ones being the β/β dimers, the heterodimers remaining with an intermediate phenotype (Aoyama *et al.,* 2004).

In addition the increase of the levels of PCho plays an important role in mitogenesis, cell proliferation and carcinogenesis. Therefore, this reduction of the intracellular levels of PCho caused by ChoKβ noverexpression could have an effect on the transformation mediated by ChoKα. For the purpose of determining the relevance of this effect, Hek293T cells were transiently transfected as has been previously described, and once the correct ectopic ChoK overexpression is verified in each case, 10⁶ cells were subcutaneously injected into each flank of athymic nu-/nu- mice (n=10-12). The mice injected with Hek293T cells transfected with ChoKα generated tumors with a rate of 25%, similar to the incidence obtained previously, whereas the mice injected with ChoKβ did not generate any tumor, remaining identical to the controls (Figure 6). Surprisingly, in the case of the cells co-transfected with both isoforms, there was a total reduction of the incidence of onset of tumors.

In addition, tumors generated in immunodepressed mice by ChoKα overexpression in these same conditions were surgically extracted, after that their cells were explanted, establishing them in culture. This cell line, called ADJ, has a constitutive activation of ChoKα and is tumorigenic in immunodepressed mice as has been recently described (Ramirez de Molina *et al.,* 2008a). For the purpose of confirming the negative effect of ChoKβ on the transforming capacity of ChoKα, ADJ cells were transfected with the ChoKβ expression vector or with an empty pCDNA3b vector as a control, after which they were injected into athymic mice as has been previously described, the tumor growth being monitored for 6.5 weeks. In the case of the ADJ cells/ChoKβ the tumors generated had a volume which was 73% smaller than the ADJ control cells transfected with the empty vector (Figure 7).

For the purpose of studying the effect of ChoKβ on cells transformed by ChoKα in greater depth, the *in vitro* proliferative capacity of the ADJ cells transfected with ChoKβ or with an empty pCDNA3b vector was studied. A proliferation experiment over time was carried out, staining the cells with crystal violet. In accordance with the results obtained *in vivo* in the athymic mice, the ADJ cells transfected with ChoKβ reflect a significant delay in the proliferation with respect to the control cells after 96 hours of maintenance in normal culture conditions (Figure 8). Taken together, these results suggest that ChoKβ plays an oncosupressor role in the transformation mediated by ChoKα.

### 2. USE OF CHOKβ AND OF THE CHOKα/CHOKβ RATIO AS A PROGNOSIS MARKER IN PATIENTS WITH CANCER

### 2.1. Materials and methods

### Patients included in the study

Frozen lung cancer tissue samples from 69 randomly selected patients who underwent surgical resection of NSCLC at La Paz University Hospital in Madrid (Spain) between 2001 and 2007 were studied. Of these 69 samples, 39 were squamous cell carcinomas, 12 were adenocarcinomas and 17 were other types of cancer. The clinical characteristics of the patients included in the study are summarized in Table 1.

### Statistical analyses

Quantification of gene expression (AQ) was calculated with the 2-^{Δct} method (Applied Biosystems) and presented as AQx10⁶. Gene expression analysis was performed using 18S endogenous gene expression for normalization.

Receiver operating characteristic (ROC) curves were obtained to show the relationship between sensitivity and false-positive rate at different cut-off values of ChoKβ expression for lung cancer-specific survival and relapse-free survival. The cut-off value was established according to the best combination of sensitivity and false-positive rate (1-specificity) based on the ROC curves.

The Kaplan-Meier method was used to estimate overall and relapse-free survival. Only death from recurrence of lung cancer was considered in the study. The effect of the different factors on tumor-related recurrence and survival was assessed by the log-rank test for univariate analysis. To assess the effect of ChoKβ expression on survival, with adjustment for potential confounding factors, proportional hazard Cox regression modeling was used. Hazard ratios (HR) and 95% confidence intervals (95% CI) were calculated from the Cox regression model. All reported p values were two-sided. Statistical significance was defined as p<0.05. Statistical analyses were done using the SPSS software (version 14.0).

**Table 1. Characteristics of the patients included in the study**

| | n (%) |
|---|---|
| Age | 43-85 years (median 66) |
| Sex | |
| Men | 61 (88.4%) |
| Women | 8 (11.6%) |
| Histology | |
| Squamous cell carcinoma | 39 (56.5%) |
| Adenocarcinoma | 12 (17.4%) |
| Others | 17 (26.1%) |
| Stage | |
| I_{A} | 6 (8.7%) |
| I_{B} | 32 (46.4%) |
| II_{A} | 2 (2.9%) |
| II_{B} | 11 (15.9%) |
| III_{A} | 9 (13%) |
| III_{B}-IV | 7 (10.1%) |
| Total | 69 |
| Relapse | |
| No | 48 (69.6%) |
| Yes | 17 (24.6%) |
| Unknown | 4 (5.8%) |

### 2.2. Prognostic value of ChoKβ expression in NSCLC

To study whether ChoKβ expression is associated with the clinical outcome of patients with NSCLC, ChoKβ gene expression was analyzed in 69 surgical samples of NSCLC using real time RT-PCR. Gene expression analysis showed that ChoKβ expression was distributed differentially in the tumors, with normalized AQ values of mRNA copies ranging between 0.42 and 30.81 (Figure 9). To establish how ChoKβ expression is in tumor samples compared with healthy tissues, ChoKβ expression was analyzed in a commercial RNA obtained from healthy human lung tissue. Most of the tumor samples showed reduced expression levels when compared to this commercial normal tissue used as a reference (normal tissue has an AQ expression of 13.89).

No relationship of ChoKβ expression with the available clinical-pathological parameters of the patients (stage, histological degree, age or sex) was found. To analyze whether the reduced ChoKβ expression observed in most of the tumors was associated with the clinical outcome of the patients, an arbitrary cut-off point of 4.022 AQ was established (70% sensitivity, 54% specificity) according to ROC methodology. Under these conditions, 35 out of the 69 (51%) tumor samples analyzed for ChoKβ expression were below this cut-off point.

Patients with reduced ChoKβ expression showed worse survival from lung cancer and relapse-free survival than those with higher concentrations of this enzyme, although these differences did not reach statistical significance (Figure 10).

The ChoKβ gene expression levels were then assessed in tumor samples from patients with stage I NSCLC (Figure 11). An association between ChoKβ expression above the cut-off point and improved lung-cancer specific survival was noted (p=0.04). Patients with reduced ChoKβ expression had a significant trend to increased risk of death compared with those with higher concentrations of the enzyme (HR 0.375 [95% CI: 0.13-1.10], p=0.07). Furthermore, a similar trend was observed when relapse-free survival was analyzed. Patients with ChoKβ expression below the cut-off point showed a significant increased risk of relapse (p=0.02), (HR 0.43 [95% CI: 0.16-1.17], p=0.1).

Similar results were obtained when ChoKβ gene expression levels were analyzed in the subset of patients with squamous cell carcinoma. Kaplan-Meier plots showed a significant trend to worse survival in those patients with low ChoKβ expression than in patients with concentrations of the enzyme above the cut-off point (p=0.08) (Figure 12). Furthermore, reduced ChoKβ expression in this type of tumor was found significantly associated to shorter relapse-free survival (p=0.03) (Figure 12).

Taken together, these results suggest that ChoKβ expression is closely associated with relapse-free and overall survival among patients with NSCLC. Multivariate Cox-regression analysis suggests that ChoKβ could be an independent factor for high risk of poorer survival for patients with reduced ChoKβ expression (HR 0.38 [95% CI: 0.13-1.11], p=0.07). In this way, ChoKβ could be a new prognostic factor that could be used to aid in identifying patients with early-stage NSCLC who might be at high risk of recurrence, and for identifying patients with favorable prognosis who could receive less aggressive treatment options or avoid adjuvant systemic treatment.

### 2.3. Combined analyses of ChoKα and ChoKβ expression as a powerful tool for NSCLC prognosis

TCD Pharma has previously demonstrated that ChoKα plays a relevant role in lung cancer, finding that the overexpression of this enzyme is an independent predictive factor of relapse-free and lung cancer-specific survival in early-stage NSCLC patients (Ramirez de Molina, A. et al., Lancet Oncol 8, 889-97 (2007). Here, the predictive value of ChoKβ expression in tumor samples of patients with NSCLC is demonstrated. These results strongly suggest that low ChoKβ expression is associated with worse clinical outcome of early-stage patients.

On the basis of these initial findings, high ChoKα expression and low ChoKβ expression were defined as two unfavorable factors that were associated with poor survival. Indeed, it was found that patients with low ChoKα and simultaneous high ChoKβ expression levels displayed the longer lung cancer-specific survival and relapse-free survival whereas the patients with high levels of ChoKα and simultaneous low levels of ChoKβ showed shorter survival (p=0.19 for overall survival and p=0.099 for relapse-free survival) (Figure 13).

Findings from Kaplan-Meier analyses for the other two combination groups: low ChoKα and simultaneous high ChoKβ and high ChoKα and simultaneous high ChoKβ expression levels, display an intermediate behavior and no differences on survival were observed between these two groups (Figure 13).

According to Cox multivariate regression analysis, patients with high levels of ChoKα and simultaneous low levels of ChoKβ showed a significant trend to be associated with lung cancer-specific death (HR of 7.8 (95% CI, 0.98 to 67.5); p = 0.06) and with recurrence of cancer (HR of 10.5 (95% CI, 1.22 to 90.0); p = 0.03). These results strongly indicate that the combined effect of the expression of both ChoK isoforms could constitute a better prognostic factor than each one separately.

### 2.4. Conclusion

The determination of ChoKβ gene expression can predict the clinical outcome in patients with NSCLC. This expression profile could be useful to improve the clinical management of NSCLC patients. Furthermore, the results presented in this report suggest that the combined effect of both ChoK isoforms provides a powerful tool for the identification of patients at high risk of recurrence and death from lung cancer in early-stage NSCLC patients.

### 3.USE OF PEMT AND/OR CHOKβ AS A MARKER OF RESPONSE TO TREATMENT WITH ChoKα INHIBITORS

### 3.1. Phosphatidylethanolamine methyltransferase (PEMT): the functional connection between ChoKα and ChoKβ

In mammals, one of the points of metabolic connection between the two branches of Kennedy's pathway for generation of PE and PC is the enzyme phosphatidylethanolamine methyltransferase (PEMT). This enzyme transforms PE by means of two successive methylations in PC (Vance & Ridgway, 1988). It has been described that this enzyme only has a relevant activity in liver cells, its contribution being 30% of the total PC content of the cell (DeLong *et al*., 1999; Li *et al.,* 2005; Reo *et al.,* 2002; Sundler & Akesson, 1975). For the purpose of determining whether this enzyme is involved in the cross-activity of ChoKα and ChoKβ, the pattern of PEMT gene expression in response to the treatment with MN58b in different cell systems was determined. To that end, Hek293T, Jurkat, H1299 and SW780 cells were treated with 20 µM MN58b and the PEMT expression was determined by means of quantitative PCR. The results are summarized in Figure 14, where is observed that in all the cases there is an increase of the levels of PEMT mRNA in response to the specific inhibition of ChoKα by MN58b.

In addition, it has been previously described that the treatment with MN58b also induces ChoKβ overexpression at transcriptional level. For the purpose of determining whether these effects are related, the PEMT expression levels have been analyzed by means of quantitative PCR in cells transfected with the ChoKβ expression vector with respect to cells transfected with an empty vector as a control. As can be observed in Figure 15, there is an induction in the PEMT expression in cells overexpressing ChoKβ, indicating that the simple overexpression of this isoform is sufficient to cause the transcriptional induction of PEMT.

## Claims

1. A method for determining the prognosis of a patient suffering from cancer comprising determining the ChoKβ expression levels in a sample of said patient, wherein reduced ChoKβ levels in relation to the levels in a reference sample are indicative of the patient showing a poor prognosis.

2. A method for determining the prognosis of a patient suffering from cancer comprising determining the ChoKα and ChoKβ expression levels in a sample of said patient, wherein reduced levels of ChoKα and high levels of ChoKβ in relation to the expression levels of said proteins in a reference sample are indicative of the patient showing a good prognosis.

3. The method according to claims 1 or 2, wherein the cancer has high ChoKα expression levels.

4. The method according to any of claims 1 to 3, wherein the cancer is lung, breast, bladder or colorectal cancer.

5. The method according to any of claims 1 to 4, wherein the prognosis is determined by means of the determination of a parameter selected from the group of survival and relapse-free survival.

6. The method according to any of claims 1 to 5, wherein the determination of the ChoKβ expression levels or of the ChoKα expression levels is carried out by means of the determination of the levels of mRNA encoded by said protein.

7. A method for determining the response of a patient with cancer to the treatment with a ChoKα inhibitor comprising determining in a sample of said patient the expression levels of a protein selected from the group of PEMT and ChoKβ, wherein an increase of the PEMT expression levels or an increase of expression of the levels of ChoKβ in relation to the levels in a reference sample are indicative of a good response to the ChoKα inhibitor.

8. The method according to claim 7, wherein the cancer is lung, breast, bladder or colorectal cancer.

9. The method according to claims 7 or 8, wherein the determination of the PEMT or ChoKβ expression levels is carried out by means of the determination of the levels of mRNA encoded by the corresponding protein.

10. A ChoKβ activity-inducing agent for its use in the treatment of cancer.

11. A ChoKβ activity-inducing agent according to claim 10, wherein the agent is selected from the group of:
(i) ChoKβ or a functionally equivalent variant of ChoKβ,
(ii) a polynucleotide encoding ChoKβ or a functionally equivalent variant thereof,
(iii)a vector comprising a polynucleotide according to (iii) and
(iv) a cell capable of secreting ChoKβ or a functionally equivalent variant thereof to the medium.

12. The composition according to claims 10 or 11, wherein the cancer is lung, breast, bladder or colorectal cancer.

13. The composition according to any of claims 10 to 12, wherein the cancer has high ChoKα expression levels.
